Europäisches Patentamt

European Patent Office

Office européen des brevets

(19)

(11) **EP 0 621 777 B1**

(12) **EUROPÄISCHE PATENTSCHRIFT**

(45) Veröffentlichungstag und Bekanntmachung des Hinweises auf die Patenterteilung:
**11.09.1996 Patentblatt 1996/37**

(21) Anmeldenummer: 93902034.3

(22) Anmeldetag: **18.01.1993**

(51) Int. Cl.6: **A61K 9/20**, A61K 9/16

(86) Internationale Anmeldenummer:
**PCT/DE93/00038**

(87) Internationale Veröffentlichungsnummer:
**WO 93/13757 (22.07.1993 Gazette 1993/18)**

(54) **WIRKSTOFF-ENTHALTENDE FESTKÖRPER MIT EINEM GERÜST AUS HYDROPHILEN MAKROMOLEKÜLEN UND VERFAHREN ZU IHRER HERSTELLUNG**

SOLID BODIES CONTAINING ACTIVE SUBSTANCES AND A STRUCTURE CONSISTING OF HYDROPHILIC MACROMOLECULES, PLUS A METHOD OF PRODUCING SUCH BODIES

CORPS SOLIDES RENFERMANT UNE MATIERE ACTIVE, A STRUCTURE COMPOSEE DE MACROMOLECULES HYDROPHILES, ET LEUR PROCEDE DE FABRICATION

(84) Benannte Vertragsstaaten:
**AT BE CH DE DK ES FR GB GR IT LI NL PT SE**

(30) Priorität: **17.01.1992 DE 4201179**
**17.01.1992 DE 4201173**
**30.04.1992 US 876864**
**30.04.1992 US 876877**

(43) Veröffentlichungstag der Anmeldung:
**02.11.1994 Patentblatt 1994/44**

(60) Teilanmeldung: **95113398.2**

(73) Patentinhaber: **ALFATEC-PHARMA GmbH**
**D-69120 Heidelberg (DE)**

(72) Erfinder:
• **WUNDERLICH, Jens-Christian**
**D-6900 Heidelberg (DE)**
• **SCHICK, Ursula**
**D-69198 Schriesheim (DE)**

• **WERRY, Jürgen**
**D-6700 Ludwigshafen (DE)**
• **FREIDENREICH, Jürgen**
**D-6905 Schriesheim (DE)**

(74) Vertreter: **KUHNEN, WACKER & PARTNER**
**Alois-Steinecker-Strasse 22**
**D-85354 Freising (DE)**

(56) Entgegenhaltungen:
EP-A- 0 081 912    EP-A- 0 351 296
EP-A- 0 412 877    EP-A- 0 450 141
WO-A-90/11757    WO-A-91/09591
FR-A- 1 259 081    GB-A- 927 218

• JOURNAL OF MICROENCAPSULATION Bd. 7, Nr. 2, 1990, Seiten 209 - 217 S.E. LEUCUTA 'CONTROLLED RELEASE OF NIFEDIPINE FROM GELATIN MICROSPHERES AND MICROCAPSULES..

## Beschreibung

Die Erfindung betrifft Pellets aus hydrophilen Makromolekülen, Wirkstoffen und gegebenenfalls weiteren pharmazeutisch akzeptablen Gerüst- und Hilfsstoffen, wobei der Wirkstoff in einer Matrix gelöst, suspendiert oder emulgiert vorliegt, und ein neues Verfahren zur Herstellung dieser Pellets, sowie weiterhin ihre Verwendung als Arzneimittel, Kosmetikum, Diagnostikum oder diätetisches Lebensmittel (health care). Als Wirkstoff werden vorzugsweise Dihydropyridinderivat, insbesondere Nifedipin, Nitrendipin oder Nisoldipin eingesetzt.

Granulate bzw. Pellets als Formkörper dienen in der pharmazeutischen Industrie hauptsächlich als Zwischenprodukte zur Tablettierung. Die Formgebung soll dabei zu einem frei fließenden, körnigen und staubfreien Produkt führen, das aufgrund seiner Gleichförmigkeit die technologische Verarbeitung und die Dosiergenauigkeit verbessert. Darüberhinaus besitzen Pellets als moderne multiple-unit-Arzneiform, beispielsweise abgefüllt in Hartgelatinekapseln, gegenüber single-unit-Arzneiformen, wie z.B. Tabletten oder Dragees, eine Reihe von Vorteilen:

- Sie verteilen sich gleichmäßig im Gastrointestinaltrakt.
- Aufgrund ihrer geringen Größe resultieren im Gegensatz zu monolithischen Arzneiformen kürzere Magenverweilzeiten, vor allem bei magensaftresistent überzogenen Arzneiformen.
- Sie lösen sich im Gastrointestinaltrakt als Einzelaggregate im Gegensatz zu einer komprimierten Tablette, die erst in ihre Granulatteilchen zerfallen muß, schneller auf.
- Es können Pellets mit unterschiedlicher Wirkstoffabgabe in gemischter Form einzeldosiert werden.

Jedoch liegt allen Verfahren des Standes der Technik die grundsätzliche Problematik der notwendigen Formgebung von pulvrig-kristallinen Wirk- und Hilfsstoffen zu verarbeitbaren Granulaten (Pellets) als Formkörper zugrunde.

Man unterscheidet dabei zwischen auf- und abbauenden Verfahren. Allen Verfahren gemeinsam ist, daß man bislang nur über mehrere und aufwendige Teilschritte zu Granulaten bzw. Pellets als Formkörper gelangt.

Bei den abbauenden Verfahren werden - vereinfacht dargestellt - zunächst die Arznei- und Hilfsstoffe zerkleinert, durch Sieben auf eine einheitliche Korngröße gebracht und dann gemischt. Danach erfolgt das trockene oder feuchte Granulieren, bei dem die Pulvermischung aggregiert und anschließend zu Granulatkörnern zerkleinert wird. Im nächsten Schritt wird, wenn nötig, getrocknet und wiederum gesiebt.

Bei den Aufbaugranulaten werden aus den pulverförmigen Arznei- und Hilfsstoffen unter kontinuierlicher Zugabe von Granulierflüssigkeit bei gleichzeitigem Trocknen Granulatkörner in einem kontrollierten Prozeß (z.B. Wirbelschichtverfahren) gebildet.

Durch anschließende, spezielle Ausrundungsverfahren (z.B. Marumerizer[R]) gelangt man zu runden, kugelförmigen Granulatteilchen (Pellets). Nachteilig hierbei ist, daß bei der Ausrundung von bereits hergestellten, unförmigen Granulatteilchen Substanzmasse mit Arzneistoff verlorengeht und nicht direkt dem Granulierprozeß wieder zugeführt werden kann. Dies stellt sicher ein kosten - und entsorgungstechnisches Problem dar. Gleichzeitig führt die mechanische Ausformung zu einem ungleichförmigen Produkt.

Spezielle Pelletiertechniken sind beispielsweise die aufbauende Trockenpelletierung durch Kompaktierung und die Wirbelschichtgranulierung, die sehr unbefriedigende Ergebnisse hinsichtlich Form und mechanischer Festigkeit der Pellets liefern.

Alle diese Herstellungsprozesse stellen technologisch aufwendige Mehrschrittverfahren dar. Sie sind gekennzeichnet durch eine Vielzahl von Prozeßparametern technologischer Art, wie z.B. Temperatur, Feuchtigkeitsgehalt, Homogenität der Mischungen u.s.w..

Weiterhin ist bei allen Granulations- und Pelletierverfahren der Einsatz einer ganzen Reihe von Hilfsstoffen notwendig. So müssen beispielsweise Bindemittel oder Granulierflüssigkeiten eingesetzt werden, um das pulverförmige Gut in eine feste, kompakte und verarbeitungsfähige Form zu bringen. Genaueste Kenntnisse um das physikalisch-chemische Verhalten z.B. Lösungswärme, Löslichkeit oder Kristallbildungstendenz und große Erfahrung im Umgang mit diesen Stoffen ist notwendig, um das Zusammenwirken dieser Hilfsstoffe untereinander und mit dem Arzneistoff im Zusammenhang mit allen zu beachtenden Prozeßparametern beurteilen zu können.

Somit können die pharmazeutischen Anforderungen an ein Granulat (Pellets) oft nur durch empirische Versuche in Abhängigkeit des zur Verarbeitung kommenden Arzneistoffs und der daraus zu formulierenden Darreichungsform erfüllt werden.

Daher wird verständlich, daß die Einhaltung konstanter Herstellungsbedingungen bei den aufwendigen Verfahren sehr schwierig ist. So ist es, bedingt durch die Vielzahl von zu beachtenden Parametern, bei den bekannten Herstellungsprozessen trotz eines hohen Entwicklungs- und Optimierungsaufwandes nicht für jeden Arzneistoff möglich, ein geeignetes Verfahren zu finden.

Betrachtet man nach dem Stand der Technik hergestellte Pellets oder Granulate darüberhinaus unter biopharmazeutischen Aspekten, so läßt sich erkennen, daß der Arzneistoff aus diesen aggregierten Formkörpern erst nach Desaggregation und anschließende Freisetzung dem Organismus zur Verfügung gestellt werden kann. Die Vielzahl prinzipiell unterschiedlicher Haft- und Bindungskräfte in Granulaten verdeutlicht diese Problematik. Durch erhärtende Bindemittel beim Trocknen (Feuchtgranulierung) oder durch Sintern bzw. Schmelzhaftung unter Druckeinwirkung (Trockengranulierung) entstehen Feststoffbrücken, deren bindende Kräfte im Organismus überwunden werden müssen, um den Arzneistoff überhaupt erst aus der Arzneiform freizugeben.

Jeder Herstellungsschritt bei den Verfahren des Standes der Technik kann somit einen ungünstigen Einfluß auf die Freisetzung des Wirkstoffs und damit auf seine Bioverfügbarkeit nehmen.

GB-A-927 218 beschreibt ein Verfahren zur Herstellung pharmazeutischer Kugeln aus Gelatine und einem pharmazeutischen Material. Zu seiner Herstellung ist das Einfrieren nicht notwendig; vom "Schockfrosten" ist in dieser Druckschrift keine Rede. EP-A-0 081 912 beschreibt die Herstellung von pharmazeutischen Dosierungsformen durch Einfüllen der Mischung aus Gerüstbildner und dispergiertem Wirkstoff in eine Form und anschließendes Gefrieren.

Dihydropyridinderivate gehören pharmakologisch gesehen zu den Calciumantagonisten. Sie sind bei einer Reihe von Herz-Kreislauferkrankungen indiziert, wie z.B. koronarer Herzkrankheit, arterieller Hypertonie, Angina pectoris usw. Die Verordnungshäufigkeit von ca. 700 Mio. definierten Tagesdosen im Jahr 1989 belegt sehr deutlich die Marktstellung dieser Stoffgruppe. Der erste Vertreter aus dieser Gruppe der Dihydropyridinderivate, das Nifedipin (1,4-Dihydro-2,6-dimethyl-4-(2-nitrophenyl)-3,5-pyridincarbonsäuredimethylester, $C_{17}H_{18}N_2O_6$) wurde inzwischen durch eine Reihe von potenten Derivaten, den sogenannten "Second-Generation-Dihydropyri nen" ergänzt, besonders Nitrendipin 1,4-Dihydro-2,6-dimethyl-4-(3-nitrophenyl)-3,5pyridincarbonsäure-ethyl-methylester, $C_{18}H_{20}N_2O_6$ und Nisoldipin Isobutyl-methyl-1,4-dihydro-2,6-dimethyl-4-(2-nitrophenyl)pyridin-3,5-dicarboxylat, $C_{20}H_{24}N_2O_6$.

Die Dosierung von handelsüblichen Nifedipin-Akut-Arzneimitteln bei Einzelgabe beträgt üblicherweise 5 - 10 mg, neuere Dihydropyridinderivate werden z.T. noch niedriger dosiert.

Um Dihydropyridine, besonders Nifedipin in eine im Organismus ausreichend schnell den Wirkstoff freisetzende Applikationsform zu bringen, sind vielfach galenische Entwicklungen vorgeschlagen worden. Diese stellen jedoch sämtlich Kompromisse dar, weil einerseits die Schwer- bzw. Unlöslichkeit dieser Wirkstoffe im physiologischen Milieu ihre schnelle Freisetzung aus Arzneiformen begrenzt bzw. erschwert. Andererseits ist die schnelle Freigabe aber Voraussetzung für einen möglichst schnellen Wirkeintritt nach der Applikation. Diese Vorgänge sind für die Steigerung der Patienten-Compliance nicht unerheblich.

Zur Zeit gebräuchliche, technologische Methoden bei der Herstellung von Akutarzneiformen von Dihydropyridinderivaten, besonders Nifedipin, sind die folgenden:

a) Verarbeitung der Wirkstoffe mit Lösungsvermittlern (Tensiden) und zusätzlich
b) Lösen der Wirkstoffe in organischen Lösungsmitteln, z.B. Polyätheralkohole des Tetrahydrofurfurylalkohols.

Wegen der bekannten Lichtempfindlichkeit der Dihydropyridine kann eine konventionelle, eingefärbte Weichgelatinekapsel z.B. als Träger (Lichtschutz) für ein oben erwähntes Nifedipin-Solubilisat bzw. eine Nifedipin-Lösung in einem organischen Lösungsmittel dienen. Nach Applikation soll das Nifedipin aus der Arzneiform in feiner Form freigesetzt werden. Dabei ist aber zu bedenken, daß der Wirkstoff danach nicht wirklich frei vorliegt, sondern zuerst aus seinem Komplex mit dem Lösungsvermittler entlassen werden muß, mit dem Nachteil daß er für den Organismus nicht ausreichend schnell verfügbar ist. Ferner ist bei diesem Vorgang auch stets das Risiko gegeben, daß Nifedipin unter physiologischen Bedingungen in gröberer kristalliner Form ausfällt, sobald kein Lösungsvermittler (Tensid) mehr wirksam ist. Darüberhinaus ist die Verwendung von Tensiden oder organischen Lösungsmitteln aus toxikologischen Erwägungen nicht unbedenklich.

Flüssige, tropffähige Nifedipin-Zubereitungen sind ebenfalls handelsüblich. Beim Patienten sind diese Nifedipin-Tropfen eine sehr beliebte Applikationsform, besonders bei älteren Patienten, die das Schlucken von festen Formlingen (Tabletten, Kapseln) als unangenehm empfinden bzw. damit Schwierigkeiten haben. Darüberhinaus besitzen sie den Vorteil der guten Dosierbarkeit.

Obwohl flüssige Arzneizubereitungen, technologisch gesehen, eigentlich gut konzipierte Akutarzneiformen darstellen (der Prozeß des Zerfalls von festen, "single-unit"-Formen wie beispielsweise Tabletten oder Kapseln entfällt) sind diese Zubereitungen im Falle der Dihydropyridine nicht zeitgemäß. Zum einen aus den bereits oben angeführten Gründen (Tensideinsatz und/oder organische Lösungsmittel), zum andern aus einem weiteren, noch weitreichenderen Grund, der in dieser Wirkstoffklasse selbst zu suchen ist. Bekanntermaßen sind Dihydropyridine stark lichtempfindlich und neigen zur Zersetzung, insbesondere in Lösungen.

Daher ist besonders beim Entnehmen von Nifedipin-Tropfen durch den Patienten aus dem Vorratsgefäß eine partielle Zersetzung des Nifedipins durch Lichtzutritt, noch vor der Einnahme, nie auszuschließen. Da diese Dosierung, besonders bei älteren Patienten ein recht zeitaufwendiger Vorgang ist, wird damit das Risiko einer Wirkstoffzersetzung vor der eigentlichen Applikation noch verstärkt.

Ferner ist zu berücksichtigen, daß selbst die Aufbewahrung von Nifedipin-Tropflösungen in braunen oder dunkel eingefärbten Glasflaschen keine ausreichende, längere Lagerstabilität (Schutz vor Lichtzutritt!) bieten kann.

Für Dihydropyridinderivate ist eine Applikation als schnell anflutende Akutform, wobei die Zubereitung selbst eine Wirkstofflösung darstellt, aus pharmakologischen Erwägungen erwünscht bzw. vorteilhaft. Jedoch kann aufgrund der physikalisch-chemischen Eigenschaften des Wirkstoffs, wie beispielsweise ungenügende Wasserlöslichkeit, Lichtempfindlichkeit in Lösung etc. diese Darreichungsform technologisch nicht oder nur unter Umwegen realisiert werden.

Die vorliegende Erfindung stellt sich die Aufgabe, neuartige Festkörper, ein Verfahren zu ihrer Herstellung sowie Mischungen vorzuschlagen, die zum einen aufgrund ihrer Struktur und Zusammensetzung die Bioverfügbarkeit und Verträglichkeit von Arzneistoffen verbessern, lagerstabil, exakt dosierbar, als single oder multiple unit vorliegen und zum andern auf umweltschonende, einfache und wirtschaftliche Weise herzustellen sind, die Wirkstoffe auf schonende Weise verarbeiten und somit insgesamt gesehen die Nachteile des Standes der Technik überwinden.

Der vorliegenden Erfindung liegt insbesondere die Aufgabe zugrunde , ein Arzneimittel für die orale bzw. perorale Applikation von Dihydropyridinderivaten, besonders Nifedipin, bereitzustellen, das für eine schnelle Arzneistofffreisetzung geeignet ist und die Probleme des Standes der Technik überwindet.

Diese Aufgabe wird durch ein Verfahren zur Herstellung von wenigstens einen Wirkstoff enthaltenden Pellets gelöst, das dadurch gekennzeichnet ist, daß man

a) einen Gerüstbildner aus hydrophilen Makromolekülen ausgewählt aus der Gruppe bestehend aus: Kollagen, Gelatine, fraktionierte Gelatine, Kollagenhydrolysate, Gelatinederivate, Pflanzenproteine, Pflanzenproteinhydrolysate, Elastinhydrolysate, in einem wäßrigen und/oder organischen Lösungsmittel löst,

b) den Wirkstoff dispergiert,

c) die erhaltene Mischung aus gelöstem Gerüstbildner und dispergiertem Wirkstoff in ein tiefkaltes inertes verflüssigtes Gas eintropft und so Pellets formt, und

d) die so geformten Pellets durch Verdampfen oder Sublimieren des Lösungsmittels auf übliche Weise trocknet.

Diese Aufgabe wird weiterhin durch ein Wirkstoff enthaltendes Pellet gelöst, welche gekennzeichnet ist durch eine Dispersion wenigstens eines Wirkstoffs oder Wirkstoffgemisches in einer Matrix, die im wesentlichen einen Gerüstbildner aus hydrophilen Makromolekülen umfaßt, welche ausgewählt worden ist aus der Gruppe bestehend aus: Kollagen, Gelatine, fraktionierte Gelatine, Kollagenhydrolysate, Gelatinederivate, Pflanzenproteine, Pflanzenproteinhydrolysate, Elastinhydrolysate und Mischungen der vorgenannten Stoffe, und welche herstellbar sind nach dem oben genannten erfindungsgemäßen Verfahren.

Unter "Pellets" werden im Sinne der Erfindung im wesentlichen symmetrisch ausgebildete Festkörper, Aggregate oder Mikropellets verstanden.

Erfindungsgemäß sind einheitlich runde Pellets, besonders für pharmazeutische Anwendungen geeignet, wobei der Begriff Pellet vorzugsweise einen Korngrößenbereich von etwa 0,2 bis 12 mm umfaßt.

In der Beschreibung der Erfindung werden die Eigenschaften, Herstellung und Verwendung anhand von runden Pellets bevorzugt dargestellt.

Jedoch kann der Fachmann auch andere Festkörper aus der Gruppe bestehend aus: Pulver, Granulate, im wesentlichen symmetrisch ausgebildete Aggregate, vorteilhaft zur Herstellung, insbesondere von Arzneiformen, einsetzen.

Als Wirkstoff wird gemäß der vorliegenden Erfindung vorzugsweise ein Dihydropyridinderivat verwendet, insbesondere Nifedipin, Nitrendipin oder Nisoldipin.

Bevorzugte Ausführungsformen der Erfindung sind in den Unteransprüchen beschrieben und beansprucht.

Die erfindungsgemäßen festen bis halbfesten oder gelförmigen Pellets stellen runde, einheitliche Formkörper im Bereich von 0,2 - 12 mm dar. Pellets im Bereich von 0,2 - 2 mm eignen sich für multiple unit dosage forms, Pellets im Bereich von 2 - 12 mm sind als single unit dosage-Form verwendbar.

Im Hinblick auf die Arzneimittelsicherheit wird von der pharmazeutischen Industrie exakte Dosiergenauigkeit, Homogenität, Verträglichkeit und Lagerstabilität der entsprechenden Arzneiform gefordert. Bei herkömmlicher Arzneimittelherstellung ist dieser Standard oft nur mit hohem und kostenintensivem Aufwand zu erreichen. Die einheitliche Korngrößenverteilung der beanspruchten Pellets, verbunden mit einer homogenen Verteilung des Arzneistoffes, verbessern gegenüber dem Stand der Technik die Dosiergenauigkeit deutlich. Weiterhin werden die in der Pelletmatrix eingebetteten Wirkstoffe in eine lagerstabile Form gebracht, die eine hohe mechanische Festigkeit mit geringem Abrieb (Friabilität) aufweist. Empfindliche Wirkstoffe werden zudem vor äußeren Einflüssen zuverlässig schützt.

Die erfindungsgemäßen Pellets als Formkörper, die sich durch ihre einheitliche runde und gleichmäßige Gestalt auszeichnen, sind aufgrund ihres harmonischen Gesamteindruckes optisch sehr ansprechend und können die Akzeptanz beim Patienten steigern. Durch entsprechende Farbgebung lassen sich die klar durchsichtigen und glänzenden, opak bis transparent oder undurchsichtig aussehenden Pellets zu unverwechselbaren Arzneispezialitäten entwickeln.

Durch die vorteilhafte Schutzkolloidfunktion der beanspruchten Makromoleküle und die gleichzeitige Einbettung der Wirkstoffe in dem polymeren Matrixgerüst wird insbesondere bei schleimhautirritierenden Wirkstoffen die Verträglichkeit deutlich erhöht. So kann z.B. die Magenschleimhautreizung bzw. Irritation von Acetylsalicylsäure durch die schleimhautprotektive Wirkung der beanspruchten Makromoleküle wirkungsvoll vermindert werden (vergl. Beispiel 7) Als Arzneiform sind die beschriebenen Pellets wohlschmeckend und peroral gut einzunehmen.

Überraschenderweise erfolgt bei allen Arzneistoffen unabhängig davon, ob sie gelöst, suspendiert oder emulgiert in den erfindungsgemäßen Pellets als Formkörper vorliegen, im Gegensatz zu herkömmlichen Granulaten, Pellets oder Tabletten die Freisetzung des aktiven Agens im Organismus ohne vorgelagerten Zerfallsprozeß. Bei herkömmlichen Zubereitungen müssen zunächst die Haft- und Bindekräfte, die eine Formgebung überhaupt ermöglichen, überwunden werden, desweiteren müssen die so erhaltenen Unteraggregate benetzt und gelöst werden, bis der Arzneistoff schließlich in einer resorptionsfähigen Form vorliegt. Herkömmliche feste Arzneiformen können je nach Art der verwendeten Hilfsstoffe und dem angewandten Herstellungsverfahren die Bioverfügbarkeit von Wirkstoffen erheblich herabsetzen.

Der Lösevorgang aus der Arzneiform als zeitbestimmender Faktor hängt bei den erfindungsgemäßen Pellets als Formkörper ausschließlich von der Art und Zusammensetzung des hydrophilen Matrixsystems ab und ist in der Freisetzungsrate modulierbar. So können Akutformen, die sich innerhalb von wenigen Sekunden auflösen, als auch Retardformen formuliert werden. Die Auflösung des Gerüstbildnders ist der geschwindigkeitsbestimmende Schritt.

Bei hydrophoben oder schwerlöslichen Arzneistoffen verbessern die beschriebenen hydrophilen Makromoleküle die Resorption bzw. die Bioverfügbarkeit und können erfindungsgemäß auf den jeweiligen Arzneistoff hinsichtlich chemisch-physikalischer und pharmazeutischer Eigenschaft abgestimmt werden.

Bei Arzneistoffen, die unter herkömmlichen Bedingungen als schlecht resorbierbar bzw. problematisch bioverfügbar gelten, kann somit durch Einarbeitung, sogar als einfache Dispersion, in eine erfindungsgemäße Zubereitung eine Bioverfügbarkeitssteigerung von bis zu 100 bis 150% erzielt werden, im Vergleich zu einer konventionellen Zubereitung gleicher Dosierung des Arzneistoffs.

Offensichtlich führt also das Vorliegen in einer erfindungsgemäßen Zubereitung zu einer stark erhöhten (effektiveren) Resorption der Arzneistoffdosis unter physiologischen Bedingungen.

Die arzneistoffhaltigen Pellets sind während des schonenden Herstellungsprozesses (Formgebung) niedrigen Temperaturen ausgesetzt und kommen nur mit einem inerten Medium (flüssiger Stickstoff) in Berührung. Daher erfolgt eine Veränderung der Arzneisstoffe oder eine Kontamination mit Rückständen von Kühlölen bzw. organischen Lösungsmitteln, wie beispielsweise von der klassischen Weichgelatinekapselherstellung bekannt ist, nicht.

Unter technologischen und biopharmazeutischen Aspekten erfüllen die beschriebenen Pellets alle prinzipiellen Anforderungen, die an diese Dosierungsform zu stellen sind:

- Sie sind in Form und Farbe gleichmäßig,
- besitzen eine enge Korngrößenverteilung,
- sind leicht dosier-und abfüllbar,
- weisen eine hohe mechanische Festigkeit und Haltbarkeit auf,
- setzen den Arzneistoff schnell oder moduliert frei.

Im Sinne der Erfindung können - allein oder in Mischungen - hydrophile Makromoleküle aus der Gruppe bestehend aus: Kollagen, Gelatine, fraktionierte Gelatine, Gelatinederivate, Kollagenhydrolysate, Pflanzenproteine Pflanzenproteinhydrolysate, Elastinhydrolysate, eingesetzt werden.

Diese biogenen Stoffe sind pharmazeutisch unbedenklich und untoxisch. Die Matrixeigenschaften der genannten Eiweißstoffe lassen sich in genauer Kenntnis ihres chemisch-physikalischen Verhaltens in weiten Grenzen einstellen und führen so zu einem Arzneimittel, bei dem der jeweilige Wirkstoff in optimaler und reproduzierbarer Form vorliegt.

Gelatine ist ein aus kollagenhaltigem Material gewonnenes Skleroprotein, das je nach Herstellungsprozeß unterschiedliche Eigenschaften hat. Sie besteht im wesentlichen aus vier Molekulargewichtsfraktionen, die die physikalisch-chemischen Eigenschaften in Abhängigkeit vom Molekulargewicht und prozentualem Gewichtsanteil beeinflussen. Je höher z.B. der Anteil Mikrogel ($10^7$ bis $10^8$ D) liegt, desto höher ist auch die Viskosität der wäßrigen Lösung. Handelsübliche Sorten enthalten bis zu 10 Gewichtsprozent. Die Fraktionen der alpha-Gelatine und deren Oligomere ($9,5 \times 10^4$ / $10^5$ bis $10^6$ D) sind entscheidend für die Gelfestigkeit und liegen üblicherweise zwischen 10 und 40 Gewichtsprozent. Molekulargewichte unterhalb der alpha-Gelatine werden als Peptide bezeichnet und können in herkömmlichen Gelatinequalitäten (niedrigbloomig) bis zu 80 Gewichtsprozent betragen.

Gelatine besitzt ein temperatur- und konzentrationsabhängiges reversibles Sol-Gel-Umwandlungsverhalten, das von der molekularen Zusammensetzung abhängig ist. Als Maß für das Gelbildungsvermögen der Gelatine ist es international gebräuchlich, die Bloomzahl anzugeben. Niedrige Handelsqualitäten beginnen bei 50 Bloom, hochbloomige Sorten liegen bei etwa 300 Bloom.

Die chemischen und physikalischen Eigenschaften variieren je nach Herstellungsverfahren, wobei besonders schonend gewonnene Gelatinesorten (geringer Anteil an rechtsdrehenden Aminosäuren und Peptiden) kurze Sol-Gel-Umwandlungsgeschwindigkeiten und Schmelzpunkte oberhalb 37°C (gemessen als 10%ige Lösung) aufweisen.

Fraktionierte Gelatine stellt den Spezialfall von Gelatine dar und wird durch spezielle Herstellungstechniken, wie z.B. Ultrafiltration aus herkömmlicher Gelatine gewonnen. Die Zusammensetzung kann z.B. durch Entfernung von Peptiden (MG < 9,5 x 10$^4$ D) oder durch Mischungen aus Einzelfraktionen wie z.B. alpha-Ketten, dimeren und trimeren Ketten oder Mikrogel variiert werden.

Darüber hinaus hat Gelatine bzw. fraktionierte Gelatine gute Tensideigenschaften mit Schutzkolloidwirkung und Emulgatoreigenschaft.

Kollagen in nativer Form ist wasserunlöslich. Durch spezielle Herstellungsverfahren gibt es heute lösliche Kollagentypen mit einem durchschnittlichen Molekulargewicht von ca. 300.000 D.

Gelatinederivate sind chemisch veränderte Gelatinen, wie z.B. succinylierte Gelatine, die z.B. für Plasmaexpander verwendet werden.

Unter Kollagenhydrolysat wird ein von Kollagen oder Gelatine druckhydrolytisch oder enzymatisch gewonnenes Produkt verstanden, das kein Sol-Gel-Umwandlungsvermögen mehr aufweist. Kollagenhydrolysate sind leicht kaltwasserlöslich und die Molekulargewichtszusammensetzung kann zwischen einigen Hundert D bis unterhalb von 9,5 x 10$^4$ D liegen. Auf enzymatischem Wege gewonnene Produkte sind in der molekularen Zusammensetzung homogener und zeigen noch gute Tensid-und Emulgatorwirkung.

Neuentwickelte Produkte stellen die Pflanzenproteine und deren Hydrolysate dar, die in ihren Eigenschaften weitgehend den Kollagenhydrolysaten entsprechen. Sie werden vorzugsweise aus Weizen und Soja gewonnen und besitzen beispielsweise Molekulargewichte von ca. 200.000-300.000 D bzw. ca. 1.000-10.000 D.

Elastinhydrolysate werden enzymatisch aus Elastin gewonnen und bestehen aus einer einzigen Polypeptidkette. Aufgrund ihres hohen Anteils an nichtpolaren Aminosäuren können sie in lipophilen Systemen verwendet werden. Elastinhydrolysate weisen ein Molekulargewicht von ca. 2.000 - 3.000 D auf und sind auf der Haut stark filmbildend.

Bei Verwendung von Pflanzenproteinen, Pflanzenproteinhydrolysaten, Elastinhydrolysaten, bzw. von Kollagenhydrolysaten (kaltwasserlösliche Gelatinen) oder Gelatinen mit einem Maximum der Molekulargewichtsverteilung von einigen Hundert D bis unterhalb von 10$^5$ D (Variante A) bildet das Trägermaterial der beanspruchten Formkörper nach einer bevorzugten Ausführungsform der Erfindung durchgeführten Lyophilisation überraschenderweise eine hochporöse und gleichzeitig mechanisch stabile Matrix aus, die sich in kaltem Wasser schnell und vollständig auflöst.

Liegt der Arzneistoff in der Matrix in gelöster oder suspendierter Form vor, sind alle aufgeführten hydrophilen Makromoleküle in den angegebenen Molekulargewichtsbereichen allein oder in Mischungen erfindungsgemäß geeignet. Emulgierte Arzneistoffe mit schneller Freisetzung werden vorteilhaft durch Verwendung von Kollagenhydrolysaten mit noch vorhandener Tensid-und Emulgatoreigenschaft hergestellt. Besonders geeignet sind enzymatisch gewonnene Hydrolysate, die ein Molekulargewicht zwischen ca. 15.000 und 20.000 D aufweisen.

Die schnelle Auflösung der beschriebenen Matrixrezepturen eignet sich für pharmazeutischen Akutformen, bei denen der Wirkstoff einzeln oder mehrfach dosiert vorliegen kann.

Zur innerlichen Anwendung lassen sich aus den erfindungsgemäßen Pellets als Formkörper vorteilhaft Instantzubereitungen formulieren. Wird z.B. der Wirkstoff in einer schnell auflösenden Matrix eingebettet und pelletiert, so erhält man lagerstabile Pellets, die man (z.B. in Beutel abgefüllt) innerhalb weniger Sekunden in kaltem Wasser vollständig auflösen kann. Erfindungsgemäß können auch hydrophile Makromoleküle mit solgel-bildenden Eigenschaften wie z.b. Gelatine und fraktionierte Gelatine geeignet sein, die ein Maximum der Molekulargewichtsverteilung oberhalb 10$^5$ D besitzen, als Gerüstsubstanzen geeignet sein.

Liegt der Arzneistoff in gelöster, suspendierter oder emulgierter Form in einer sol-gel-bildenden Gerüstmatrix (Variante B) wie Gelatine oder fraktionierte Gelatine vor, so erhält man Pellets, die den Wirkstoff - je nach molekularer Zusammensetzung der verwendeten Gelatinesorte - schnell oder langsam in wäßrigem Medium bei 37°C freisetzen.

In einer weiteren Ausführungsform der Erfindung können Zusätze von Weichmachern von 1-50% (bezogen auf die zu verarbeitende Masse) ausgewählt aus der Gruppe bestehend aus: Glycerol, Propylenglykol, Polyethylenglykole, Triacetin, Sorbitol, Sorbitangemische, Sorbitollösungen, Glucosesirup und andere Polyole bzw. Zuckeralkohole, geeignet sein. Die genannten Stoffe beeinflussen die erfindungsgemäße Matrix hinsichtlich der Konsistenz von fest bis halbfest oder gelförmig, ihr Auflöseverhalten und die Viskosität. Besonders vorteilhaft eignet sich als Weichmacher Sorbitol, der als Süßstoff mit nicht-kariogener Eigenschaft zugleich als Geschmackskorrigens dient.

In einer besonderen Ausführungsform der Erfindung weisen Pellets aus Matrixmassen mit Weichmacherzusätzen von 20 bis 50% (bezogen auf die zu verarbeitende Masse) ausgeprägte bioadhäsive Eigenschaften auf.

Weiterhin kann es erwünscht sein, den beschriebenen Matrixmassen lipophile Bestandteile, wie z.B. Phospholipidezur Ausbildung von Liposomen zuzusetzen.

Für Pellets als Formkörper, die sich in Wasser bei 37°C innerhalb weniger Minuten auflösen, werden bevorzugt Gelatinesorten ausgewählt, deren Peptidanteil oberhalb 30% liegt und die ein Maximum der Molekulargewichtsverteilung bei ca. 10$^5$ D bis 10$^6$D aufweisen.

Für die Formulierung von Pellets mit retardierenden Eigenschaften sind im Sinne der Erfindung Gelatinesorten mit einem Peptidanteil unterhalb 10% und einem Mikrogelanteil von 10-15% geeignet. So aufgebaute Matrixmassen bzw. Mischungen besitzen in wäßriger Lösung einen Schmelzbereich von 35°C bis 40°C, bevorzugt oberhalb 37°C. Ein Zusatz von Weichmachern kann im Bereich zwischen 1 und 30% (bezogen auf die zu verarbeitende Masse) liegen. Als zusätzliche Gerüstbildner von 1-50% (bezogen auf die zu verarbeitende Masse) können eingesetzt werden: Albumine, Agar-Agar, Gummi arabicum, Pektine, Traganth, Xanthan, natürliche sowie modifizierte Stärken, Dextrane, Dextrine, Maltodextrin, Chitosan, Alginate, Alginat-Calciumphosphate, Cellulosederivate, Polyvinylalkohol, Polyvinylpyrrolidon, Polyacrylsäure und Polymere aus Methacrylsäure und Methacrylsäureestern.

Celluloseacetatphtalat oder Hydroxypropylmethylcellulosephtalat, azo-vernetzte Polymethacrylate; Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden; Galactomannanderivate wie Ethyl- oder Acetylgalactomannane; mit Adipinsäure vernetzte Polysaccharide, lipophile Substanzen wie abbaubare Mono-, Di-, und Triglyceride; erodierbare Fettalkohole.

In einer weiteren Ausführungsform der Erfindung können 1-50 % ige Zusätze von Stoffen aus dieser Gruppe ausgewählt werden, um die physikalischen oder chemischen Eigenschaften der Matrix wie z.B. die Viskosität, die mechanische Festigkeit oder die Auflöseeigenschaften des polymeren Gerüstes auf den Wirkstoff und den Anwendungszweck abzustimmen. So können beispielsweise Stoffe wie Dextrane, modifizierte Stärken, Zucker und insbesondere Mannit erfindungsgemäß Pellets hergestellt werden, die als Lyophilisat ein hochporöses Netzwerk ausbilden. Makromoleküle wie z.B. Alginate, Agar-Agar, Pektine können erfindungsgemäß zur zusätzlichen Verzögerung oder Modifizierung der Wirkstoffreigabe dienen.

Zu dieser Grundmasse können weitere, für pharmazeutische Anwendung geeignete Hilfs- und Trägerstoffe, wie z.B. Füllstoffe, wie z.B. Lactose, Dispergiermittel, wie z.B. Dinatriumphosphat, pH-Korrigentien, wie z.B. Dinatriumcitrat, Emulgatoren, wie z.B. Lecithin, Stabilisatoren, wie z.B. Ascorbinsäure, Kosolventien, wie z.B. Polyethylenglycol, natürliche Farbstoffe, wie z.B. Carotinoide, aromatisierende Stoffe oder Geschmackskorrigentien, wie z.B. Zuckerersatzstoffe, Komplexbildner oder Einschlußkomplexbildner, wie z.B Cyclodextrin zugesetzt werden.

In einer besonderen Ausführungsform der in Variante A und B angegebenen Matrixmassen bzw. Mischungen, die mit oder ohne Weichmacherzusatz aufgebaut sein können, kann durch Zusatz von magensaftresistenten Stoffen aus der Gruppe: Poly- und Methacrylsäurederivate, Cellulosederivate, und deren Mischungen Pellets hergestellt werden, die den Arzneistoff erst nach der Magenpassage freisetzen, d.h. daß der Gerüstbildner der Matrix-Mischung sich in einem vorbestimmten pH-Bereich auflöst.

Anstatt der obengenannten magensaftresistenten Stoffe können auch Substanzen verwendet werden, die erst nach Erreichen eines bestimmten Darmabschnittes durch dort vorhandene Enzyme abgebaut werden. Hierbei handelt es sich z.B. um azo-vernetzte Polymethacrylate; Polyurethan-Zucker-Copolymere, wobei als Zuckerkomponente insbesondere oligomere Galactomannane oder Galactomannandeerivate geeignet sind, die dann mit aliphatischen Diisocyanaten vernetzt werden; Galactomannanderivate wie Ethyl- oder Acetylgalactomannane; mit Adipinsäure vernetzte Polysaccharide.

Auf diese Weise sind erfindungsgemäß Pellets herstellbar, die sich insbesondere für Colonarzneiformen eignen. Nach Erreichen des Colons wird eine derartige Pelletmatrix enzymatisch abgebaut und der inkorporierte Arzneistoff damit gezielt in diesem gastrointestinalen Abschnitt freigegeben.

Weitere Ausführungen zu Colonararzneiformen finden sich insbesondere in der "Peptidarzneistoffe enthaltende Formkörper und ihre Herstellung sowie deren Verwendung" betitelten internationalen PCT-Anmeldung der ALFATEC-Pharma GmbH vom gleichen Tage.

Im Falle von alginathaltigen Grundrezepturen kann man durch Suspendierung von wasserunlöslichem Dicalciumhydrogenphosphat $[(Ca_2(HPO_4)_2]$ z.B. zu einer pH-neutralen bis leicht basischen Gelatine/Alginat-Mischung Pellets erzeugen, die den Wirkstoff verzögert freigeben. Während der Magenpassage löst das saure Medium das Calciumsalz auf und vernetzt das Alginat.

Weiterhin können erfindungsgemäß zu den von Kollagen abgeleiteten Gerüststoffen pharmazeutisch akzeptable Härtungsmittel, wie z.B. Aldosen oder Citral zugegeben werden, die nach der Trocknung zu einer Vernetzung führen.

Als Härtungsmittel ist insbesondere Xylose geeignet, da sie eine gezielt steuerbare Vernetzung der Pelletmatrix ermöglicht. Auf diese Weise können Retardarzneiformen, sogenannte Sustained-release Arzneiformen, realisiert werden, wobei erfindungsgemäß unterschiedliche Freigabecharakteristiken des Arzneistoffs mit hoher Reproduzierbarkeit eingestellt werden können.

Diese Modulierung der Arzneistofffreigabe zeigt sich besonders deutlich, wenn man das Verhalten einer derartig vernetzten Matrix in wäßrigem Milieu betrachtet. Die Pellets lösen sich im wäßrigen Milieu nicht mehr auf, sie zeigen vielmehr aufgrund der Vernetzung (Derivatisierung des Gerüstbildners) ein mehr oder weniger stark ausgeprägtes Quellungsverhalten. Dieses Quellungsverhalten ist nun über die Menge an zugesetztem Vernetungsagens, d.h. durch das Ausmaß der Härtung bzw. über die gewählten Härtungsbedingungen kontrolliert einstellbar. Dabei lassen sich ganz gezielt und mit hoher Reproduzierbarkeit unterschiedliche molekulare Fraktionen eines von Kollagen abgeleiteten Gerüstbildners vernetzen.

Einerseits können somit erfindungsgemäß Arzneistoff-Freigabeprofile erzielt werden,, die der herkömmlichen Diffusion aus Matrixformulierungen entsprechen (Quadratwurzelgesetz, vergleiche Higuchi-Gleichung).

Andererseits aber, was umso erstaunlicher ist, kann unter Verwendung derselben Ausgangsmaterialien (Gerüststoffe und Vernetzungsmittel) ebenso ein Arzneistoff-Freigabeprofil nullter Ordnung (lineare Kinetik) reproduzierbar eingestellt werden. In diesem speziellen Fall kann man eine Nicht-Fick'sche Diffusion aus der Matrix annehmen, d.h. eine quellungskontrollierte Diffusion mit einem Übergang einer glasartigen Matrix in eine gequollene Matrix, wobei der Diffusionskoeffizient des Arzneistoffs in der Matrix selbst nach und nach während des Quellungsvorgangs ansteigt. Ebenso lassen sich Zwischenzustände zwischen beiden aufgezeigten Freigabeprofilen einstellen.

Da die die erfindungsgemäßen Pellets, als Formkörper, hohe mechanische Stabilität besitzen, können sie mit pharmazeutisch gebräuchlichen Filmbildnern überzogen werden. Besonders vorteilhaft kann durch Kombination von Matrixmassen, die insbesondere bioadhäsive Eigenschaften aufweisen, und Filmüberzügen (z.B. Poly-und Methacrylsäurederivate), die sich in definierten pH-Bereichen auflösen, gezielt der gewünschte Resorptionsabschnitt im gastrointestinalen Trakt erreicht werden.

Solche bioadhäsiven Eigenschaften können beispielsweise durch Teilvernetzung einer Matrix erzeugt werden, die aus einem von Kollagen abgeleiteten Hilfsstoff aufgebaut ist.

Statt Eudragiten[R] können auch geeignete Filmüberzüge aus Substanzen, die nach Erreichen des Colons durch dort vorhandene Enzyme abgebaut werden, eingesetzt werden. Hierbei handelt es sich z.B. um azo-vernetzte Polymethacrylate; Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden; Galactomannanderivate wie Ethyl- oder Acetylgalactomannane; mit Adipinsäure vernetzte Polysaccharide.

Diese Vorgehensweise ermöglicht es, Arzneistoffe mit problematischer Bioverfügbarkeit kontrolliert zur Resorption zu bringen. Weiterhin können durch Kombinationen von Filmbildnern Pelletmischungen erfindungsgemäß hergestellt werden, die den Wirkstoff aus der Arzneiform gepulst freisetzen.

Die bereits erwähnte, erfindungsgemäß erzielbare Bioverfügbarkeitssteigerung ist überraschenderweise ebenso bei der kontrollierten Vernetzung einer Pelletmatrix gegeben. Somit lassen sich erfindungsgemäß vorteilhaft Pelletarzneiformen mit modulierter bzw. gepulster Arzneistoff-Freigabe unter Erhalt der gesteigerten Bioverfügbarkeit des Arzneistoffs herstellen.

Bekanntermaßen besitzt Gelatine je nach Herstellungsverfahren einen isoelektrischen Punkt im sauren (Gelatine Typ B) oder im alkalischen Bereich (Gelatine Typ A). Diese Eigenschaft wird erfindungsgemäß zur direkten Bildung von Mikro- bzw. Nanokapseln in der Matrixmasse ausgenutzt. So können bei Verwendung von Gelatinen mit entgegengesetzter Ladung in Mischung mit wirkstoffhaltiger Lösung (z.B. bei einem pH von 6-7) durch Entfernung des Lösungsmittels Mikrokapseln hergestellt werden. Bei Verwendung von Gelatinesorten oder Kollagenderivaten mit definierter molekularer Zusammensetzung lassen sich dreidimensionale Vernetzungen im Nanometerbereich durchführen. Gelatinen oder Kollagenhydrolysate können weiterhin mit dem Wirkstoff z.B. unter einem ca. 2-3%igen Zusatz von Salzen Konjugate bilden.

Die eingangs beschriebene, erfindungsgemäße Bioverfügbarkeitssteigerung von Arzneistoffen läßt sich erstaunlicherweise bereits erzielen, wenn ein Arzneistoff in grobdisperser Form in einer Pelletmatrix dispergiert vorliegt.

Bei Verwendung von mikronisierten Pulvern, die in einer erfindungsgemäßen Pelletmatrix dispergiert vorliegen, ergibt sich im Vergleich zu einer herkömmlichen Suspension eines mikronisierten Pulvers nochmals eine deutliche Bioverfügbarkeitssteigerung. So wird in Beispiel 8 eine Akutarzneiform auf Pelletbasis beschrieben, die Ibuprofen enthält. Die Bioverfügbarkeit dieser Pelletzubereitung gegenüber einer herkömmlichen, peroral applizierten Suspension von mikronisiertem Ibuprofen ist bei gleicher Dosierung um ca. 100% bis 150% gesteigert. Offensichtlich führt das Vorliegen eines Arzneistoffs in einer erfindungsgemäßen Zubereitung vorteilhafterweise zu einer sehr stark erhöhten (effektiveren) Resorption des Arzneistoffs unter physiologischen Bedingungen.

Wirkstoffe mit problematischer Bioverfügbarkeit lassen sich erfindungsgemäß in einer weiteren Ausbildungsform durch direkte und kontrollierte Ausfällung des vorher in der Matrixmasse gelöst vorliegenden Wirkstoffes z.B. durch pH-Verschiebung oder Entfernung des Lösungsmittels in eine feindisperse, die Resorption fördernde Form bringen.

Als besonders feindisperse Arzneistoffverteilungen sind kolloid-disperse Arzneistoffsysteme (Nanosole) geeignet, deren Eigenschaften und Herstellung in zahlreichen Patentanmeldungen der ALFATEC-Pharma GmbH beschrieben sind (z.B. PCT-Anmeldung PCT/DE92/01010 und dort zitierte weitere PCT-Anmeldungen).

Pharmazeutisch übliche organische Lösungsmittel und Kosolventien, die bevorzugt in wäßriger Lösung mischbar sind, können den beanspruchten Matrixmassen, sofern der Wirkstoff wasserunlöslich ist, zugesetzt werden.

Durch Kombination von Pellets, die Wirkstoffe aus unterschiedlichen Indikationsgruppen enthalten, lassen sich Kombinationspräparate erhalten, z.B. durch Abfüllen in übliche Hartgelatinekapseln. Sinnvolle Kombinationen können beispielsweise sein:

Dihydropyridinderivat + beta-Sympathicolyticum oder Diureticum.

Andere Anwendungszwecke sind z.B. das Abfüllen in Beutel zu Trinkgranulaten (-pellets) oder die Verwendung zur Bereitstellung von Initialdosen in Retardarzneiformen etc.

Von einem einzigen Produkt - den erfindungsgemäßen Formkörpern - ausgehend ist damit eine beträchtliche technologische Anwendungsbreite gegeben.

Im folgenden wird das Verfahren zur Herstellung der erfindungsgemäßen Pellets näher beschrieben. Weitere Ausführungen hierzu sind in den im folgenden aufgelisteten parallelen internationalen (PCT)-Anmeldungen enthalten. Die Inhalte dieser parallelen PCT-Anmeldungen, am selben Tage beim Deutschen Patentamt von denselben Erfindern und Anmeldern eingereicht:

PCT/DE93/00037

PCT/DE93/00035 und
PCT/DE93/00036
werden hiermit ebenso vollinhaltlich zur Offenbarung der vorliegenden Anmeldung gemacht, wie die älteren PCT-Anmeldungen:
PCT/DE92/01010, PCT/DE92/01012, PCT/DE92/01014, PCT/DE92/01016, PCT/DE92/01007, PCT/DE92/01008, PCT/DE92/01015, PCT/DE92/01013, PCT/DE92/01009, PCT/DE92/01011 vom 4.12.1992.

Im einfachsten Fall läßt sich das erfindungsgemäße Verfahren zur Herstellung von Wirkstoff-enthaltenden Pellets mit den folgenden drei Verfahrensschritten beschreiben:

a) Man löst einen Gerüstbildner aus hydrophilen Makromolekülen in einem Lösungsmittel,

b) man dispergiert in dieser Lösung den Wirkstoff und

c) man tropft die erhaltene Mischung aus gelöstem Gerüstbildner und dispergiertem Wirkstoff in ein tiefkaltes inertes verflüssigtes Gas ein und bildet damit den Festkörper aus.

Der erste Schritt des Verfahrens besteht darin, daß man das hydrophile Makromolekül, insbesondere Gelatine, fraktionierte Gelatine, Kollagenhydrolysate oder Gelatinederivate oder auch Mischungen von makromolekularen Stoffen in einem geeigneten Lösungsmittel - Wasser als Lösungsmittel der Wahl ist in den meisten Fällen zu bevorzugen - löst. Dabei kann Anwendung von Wärme erforderlich sein, wie z.B. bei Gelatine eine Temperatur von 37°C oder mehr, um ein Gelatinesol zu erhalten.

Weitere Hilfs- und Trägerstoffe, wie z.B. Füllstoffe, wie z.B. Lactose, Dispergiermittel, wie z.B. Dinatriumhydrogenphosphat, pH-Korrigentien, wie z.B. Dinatriumcitrat, Emulgatoren, wie z.B. Lecithin, Stabilisatoren, wie z.B. Ascorbinsäure, Kosolventien, wie z.B. Polyethylenglycol, natürliche Farbstoffe, wie z.B. Carotinoide, aromatisierende Stoffe oder Geschmackskorrigentien, wie z.B. Zuckerersatzstoffe, Komplexbildner oder Einschlußkomplexbildner, wie z.B Cyclodextrin zugesetzt werden.

Konzentrationsbereiche der hydrophilen Makromoleküle, insbesondere Gelatine, Kollagenhydrolysate oder Gelatinederivate liegen bevorzugt unterhalb von 30% (Gewichtsprozent), z.B. im Bereich von 3 - 15%, bezogen auf die zu verarbeitende Masse ohne Wirkstoff. Entsprechend beträgt der Wassergehalt der zu verarbeitenden Masse bis zu ca. 70 Gew.-% oder mehr.

Konzentrationsbereiche der zusätzlichen Gerüstbildner, wie beispielsweise Dextrane, Saccharose, Glycin, Lactose, Polyvinylpyrrolidon, insbesondere aber Mannit liegen unterhalb von 30% (Gewichtsprozent), z.B. im Bereich von 0 - 15%, bezogen auf die zu verarbeitende Masse ohne Wirkstoff. Vorzugsweise ist der Anteil an zusätzlichem Gerüstbildner nicht größer als der Anteil an dem eigentlichen Gerüstbildner.

Diese Stoffe, insbesondere aber Mannit, können als Füllkomponenten die Stabilität des polymeren Gerüsts in den erfindungsgemäßen Pellets verbessern und somit auch deren mechanische Eigenschaften.

Im zweiten Schritt wird das Dihydropyridinderivat in möglichst feinteiliger Form in der Lösung des hydrophilen Makromoleküls dispergiert.

Das im zweiten Schritt beschriebene System wird nun im dritten Schritt zur Formgebung über ein geeignetes Dosiersystem in eine tiefkalte, leicht verdampfbare Flüssigkeit eingetropft, bevorzugt in ein Tauchbad mit flüssigem Stickstoff. Jeder diskrete Tropfen nimmt dabei einerseits bereits während des freien Falls, andererseits im Tauchbad durch die um ihn entstehende Gashülle bzw. die Grenzflächenspannung System/Gas Kugelgestalt an, bevor ein vollständiges Ausfrieren erfolgt. Gerade dieses schnelle, aber dennoch kontrolliert steuerbare Gefrieren fixiert den gegebenen Zustand des Systems augenblicklich, d.h. es kann kein Arzneistoff in das umgebende Medium diffundieren, gelöster Arzneistoff kann nicht mehr auskristallisieren, Suspensionen können nicht mehr sedimentieren, Emulsionen können nicht mehr brechen, thermisch empfindliche oder feuchtigkeitsempfindliche Stoffe werden cryokonserviert, das Trägergerüst kann nicht zusammenschrumpfen usw. Das Herstellungsverfahren mit einem inerten Flüssiggas hat also keine nachteilige Beeinflussung oder Veränderung des Produkts zur Folge, was einen großen Vorteil darstellt. Die gewünschten Eigenschaften werden beibehalten.

Bei einer Ausführungsform des unter a) beschriebenen Verfahrensschrittes stellt man eine tropffähige Masse, vorwiegend aus hydrophilen Makromolekülen als Gerüstbildner, insbesondere Pflanzenproteine, Pflanzenproteinhydrolysate, Kollagen, Gelatine, fraktionierter Gelatine, Elastinhydrolysate, Kollagenhydrolysaten, Gelatinederivaten oder Mischungen der vorgenannten Stoffe und dem Wirkstoff her.

Zunächst dispergiert, d.h. löst, suspendiert oder emulgiert man den Wirkstoff, z.B. in dem gelöst vorliegenden Gerüstbildner, insbesondere Pflanzenproteine, Pflanzenproteinhydrolysate, Kollagen, Gelatine, fraktionierte Gelatine, Gelatinederivate, Kollagenhydrolysate oder Elastinhydrolysat, wobei die Art und Menge des eingesetzten Gerüstbildners und gegebenenfalls der Zusatz von weiteren Hilfsstoffen vom späteren Verwendungszweck der Formkörper abhängt. Die Konzentration des Trägermaterials kann beispielsweise von 0,5 bis 60% (g/g), bevorzugt 0,5 bis 30% (bezogen auf die zu verarbeitende Masse) variieren. Die Anwendung von Wärme im Temperaturbereich von ca. 30°C bis 60°C, vorzugsweise ca. 45°C, kann z.B. beim Einsatz von Gelatine erforderlich sein, um diese in die Solform zu überführen.

Ein Zusatz von zusätzlichen Gerüstbildnern von 1-50% (bezogen auf die zu verarbeitende Masse) ausgewählt aus der Gruppe bestehend aus: Albuminen, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyacrylsäure und Polymere aus Methacrylsäure und Methacrylsäureestern, Celluloseacetatphtalat oder Hydroxypropylmethylcellulosephthalat, azo-vernetzte Polymethacrylate; Polyurethan-Zucker-Copolymere, wobei als Zuckerkomponente insbesondere oligomere Galactomannane oder Galactomannanderivate geeignet sind, die dann im aliphatischen Diisocyanaten vernetzt werden; Galactomannanderivate wie Ethyl- oder Acetylgalactomannane; mit Adipinsäure vernetzte Polysaccharide; lipophile Substanzen wie abbaubare Mono-, Di-, und Triglyceride; erodierbare Fettalkohole kann weiterhin der Matrixmasse zugegeben werden.

In einer weiteren Verfahrensvariante können der Matrix Zusätze von Weichmachern von 1-50% (bezogen auf die zu verarbeitende Masse) ausgewählt aus der Gruppe bestehend aus: Glycerol, Propylenglykol, Polyethylenglykole, Triacetin, Sorbitol, Sorbitangemische, Sorbitollösungen, Glucosesirup und andere Polyole bzw. Zuckeralkohole beigefügt sein.

Zu dieser Grundmasse können weitere für pharmazeutische Anwendung geeignete Hilfs- und Trägerstoffe, wie z.B. Füllstoffe, wie z.B. Lactose, Dispergiermittel, wie z.B. Dinatriumhydrogenphosphat, pH-Korrigentien, wie z.B. Dinatriumcitrat, Emulgatoren, wie z.B. Lecithin, Stabilisatoren, wie z.B. Ascorbinsäure, Kosolventien, wie z.B. Polyethylenglycol, natürliche Farbstoffe, wie z.B. Carotinoide, aromatisierende Stoffe oder Geschmackskorrigentien, wie z.B. Zuckerersatzstoffe, Komplexbildner oder Einschlußkomplexbildner, wie z.B Cyclodextrin zugesetzt werden.

Selbstverständlich eignen sich die erfindungsgemäßen Mischungen zu einer sofortigen Abfüllung in flüssiger Form nach dem unter a) beschriebenen Verfahrensschritt zur Ausformung in Behältnissen, wie z. B. Formen, Weichgelatinekapseln sowie geeignete andere Umhüllungen.

In einer Ausführungsform des unter b) beschriebenen Verfahrensschrittes wird die beschriebene Matrixmasse zur Ausrundung (Formgebung) und Schockfrostung in ein Tauchbad im Bereich von ca. -70°C bis ca. -270°C, vorzugsweise von ca. - 100°C bis -220°C eingetropft. Als tiefkalte, insbesondere inerte, Flüssigkeit wird vorzugsweise flüssiger Stickstoff eingesetzt, der die Bestandteile der Pellets nicht verändert. In der tiefkalten Flüssigkeit bilden sich runde Formkörper (Pellets), die nach der Trocknung eine mechanisch stabile Matrix ausbilden. Die Formgebung erfolgt über ein geeignetes Dosiersystem. Jeder diskrete Tropfen nimmt dabei einerseits bereits während des freien Falls, andererseits im Tauchbad durch die um ihn entstehende Gashülle bzw. die Grenzflächenspannung System/Gas Kugelgestalt an, bevor ein vollständiges Ausfrieren erfolgt. Gerade dieses schnelle, aber dennoch kontrolliert steuerbare Gefrieren fixiert den gegebenen Zustand des Systems augenblicklich, d.h. es können keine Wirkstoffe in das umgebende Medium diffundieren, gelöste Bestandteile können nicht mehr auskristallisieren, Suspensionen können nicht mehr sedimentieren, Emulsionen können nicht mehr brechen, thermisch empfindliche oder feuchtigkeitsempfindliche Wirkstoffe werden cryokonserviert, das Trägergerüst kann nicht zusammenschrumpfen usw. Das Herstellungsverfahren mit einem inerten Flüssiggas hat also keine nachteilige Beeinflussung oder Veränderung des Wirkstoffes oder der Matrixmasse zur Folge. Von besonderem Vorteil ist somit der Erhalt der gewünschten Eigenschaften. Weiterhin arbeitet das Verfahren lösungsmittelfrei, belastet die Umwelt nicht und kann unter Sterilbedingungen durchgeführt werden.

Als Dosiersysteme eignen sich alle Vorrichtungen, die diskrete, gleichmäßige Gebilde, z. B. Tropfen, vorherbestimmbarer Größe erzeugen können.

Verwendet man z.B. ungeregelte Tropfvorrichtungen, so erhält man Granulate, bei Verwendung von geeigneten Sprüh- oder Zerstäubungsdüsen mit Dosierpumpen erhält man bevorzugt Pulver als Formkörper.

Weiterhin können für das erfindungsgemäße Verfahren Dosiervorrichtungen mit Düsen, die das zu tropfende Gut getaktet oder intermittierend ausstoßen, verwendet werden.

Eine weiterhin bevorzugte Ausführungsform des erfindungsgemäßen Verfahrens setzt das von Messer Griesheim GmbH entwickelte Cryopel«-Verfahren (basierend auf DE-OS 37 11 169) ein. In Verbindung mit einer Tauchfrostanlage, der Cryopel$^R$-Anlage, ist die apparative Umsetzung des erfindungsgemäßen Verfahrens in den industriellen Maßstab besonders einfach. Diese Anlage, die mit flüssigem Stickstoff betrieben werden kann, zeichnet sich besonders durch ihre Wirtschaftlichkeit aus. Diese Anlage ist auch für Sterilherstellung geeignet. Kontinuierliche Arbeitsweise bei geringem Wartungs- und Reinigungsaufwand ermöglicht die wirtschaftliche Umsetzung des erfindungsgemäßen Verfahrens in den industriellen Maßstab.

Es zeigt:

Fig. 1: eine schematische Darstellung in Schnittansicht einer Vorrichtung zur Ausführung des erfindungsgemäßen Verfahrens; und

Fig. 2: eine weitere Ausführungsform einer Vorrichtung zur Durchführung des erfindungsgemäßen Verfahrens in schematischer Darstellung.

Fig. 3: schematisch die Vorgänge, die bei der passiven Resorption von Arzneistoffen in der Gastrointestinalmembran ablaufen.

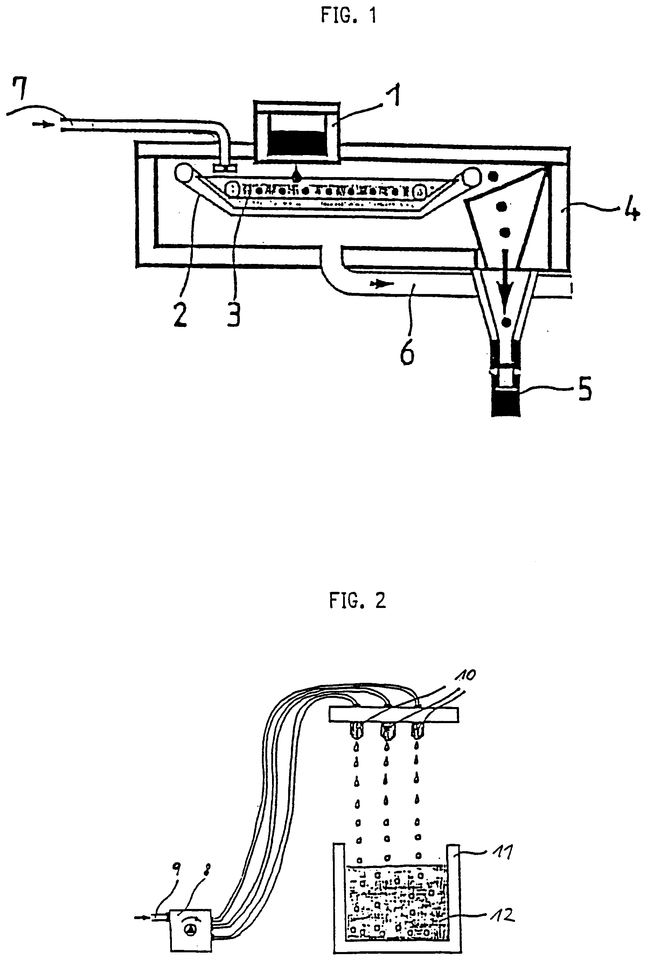

In Fig. 1 ist das von Messer Griesheim GmbH entwickelte Cryopel[R]-Verfahren schematisch dargestellt. Die erfindungsgemäße Matrixlösung, die den Wirkstoff gelöst, emulgiert oder suspendiert enthält, wird aus der beheizbaren Eintragsvorrichtung 1 über kalibrierte Düsen in das Flüssigstickstoffbad 3 bei -196°C eingetropft und unter gleichzeitiger Schockfrostung zu runden Pellets geformt. Über das kontinuierlich über Umlenkrollen laufende Transportband 2 wird das gefrorene Produkt über die Vorrichtung 5 ausgetragen. Die Dosierung des Flüssigstickstoffes erfolgt über die Zuleitung 7 und das entstehende Stickstoffgas entweicht über die Leitung 6. Die Isolierung 4 umschließt das gesamte System.

In Fig. 2 ist eine schematische Darstellung eines Verfahrens gezeigt, bei der über eine regelbare Dosierpumpe 8 die kalte bzw. auf max. 60°C erwärmte Wirkstoff-Matrixdispersion über die Zuleitung 9 kontinuierlich über die beheizbaren Tropfdüsen 10 in die Isolierwanne 11 mit Flüssigstickstoff 12 eintropft. Die schockgefrosteten Pellets werden chargenweise oder kontinuierlich entnommen. Mit dieser Vorrichtung lassen sich hochviskose Massen verarbeiten.

Sollte das zu verarbeitende System nicht ausreichend fließ- bzw. tropffähig sein, kann z.B. weiterer Wasserzusatz von 1-10 Gew.% erfolgen, die Verarbeitungstemperatur erhöht werden oder aber auch Druck bei der Dosierung zur Anwendung kommen. Im umgekehrten Falle (System zu niedrigviskos) ist analog Unterdruck bzw. Temperaturerniedrigung anzuwenden. Auf diese Weise gewährleistet man gleichmäßige Bildung, wie auch Abriß der einzelnen Tropfen.

Die Verarbeitungstemperatur kann in weiten Bereichen variiert werden, soll aber im Falle von thermolabilen Wirkstoffen unterhalb von 50°C liegen.

Somit können beispielsweise mit den beschriebenen Dosiervorrichtungen Massen, deren Viskosität sich in einem weiten Bereich bewegt, z.B. $1\times10^{-3}$ bis 12,5 Pa x s (Pascalsekunden) und höher, problemlos dosiert werden.

Weitere tiefkalte inerte verflüssigte Gase, die sich für das erfindungsgemäße Verfahren eignen, können z.B. flüssige Edelgase wie Argon sein.

In Abhängigkeit vom gewählten Dosiersystem kann eine Korngrößeneinheitlichkeit von über 80% erreicht werden, die sich durch Klassieren noch zusätzlich erhöhen läßt.

Durch Klassieren der gefrorenen und abgetrennte Anteile können diese erneut in den flüssigen Zustand überführt und wieder pelletiert werden, so daß ein verlustfreies Arbeiten gewährleistet ist.

In einer bevorzugten Ausführungsform der Erfindung werden die Pellets getrocknet, wobei sich zwei Verfahrensvarianten ergeben.

Verfahrensvariante A:

Die bei -196°C (flüssiger Stickstoff) gefrorenen Formkörper, z.B. Pellets, werden in eine Gefriertrocknungsanlage überführt. Dabei werden Temperaturen von 15°C unterhalb des Sublimationspunktes von Wasser bei einem Druck von 0,1 Pa bis $10^3$ Pa (0,001 bis 1,03 mbar) gewählt. Der Trocknungsvorgang, der in einer herkömmlichen Gefriertrocknungsanlage (Kondensatortemperatur -40°C) bei -25°C und 33 Pa (0,33 mbar) in der Primärtrocknung unter Sublimation des durch die Schockfrostung amorph gefrorenen Wassers aus der Matrix abläuft, führt nach Sekundärtrocknung (Desorption) zu einem Endprodukt mit einem hochporösen Netzwerk. Durch die erfindungsgemäße Schockfrostung wird das Wasser überwiegend an der Ausbildung einer kristallinen Phase gehindert, wodurch sich eine feste feindisperse amorphe Wasserphase in der Matrix ausbildet. Nach der Sublimation des derartig vorliegenden Wassers entstehen hochporöse Mikroporen-haltige Netzwerke, die gegenüber herkömmlichen Gefrierverfahren eine deutlich erhöhte Oberfläche aufweisen. Solche Pellets sind gegenüber herkömmlich gefriergetrockneter Ware besonders leicht löslich und sind bevorzugt zur Entwicklung von Instantzubereitungen geeignet.

Verfahrensvariante B:

Die gefrorenen Formkörper, z.B. Pellets, werden aufgetaut und konventionell getrocknet. Hierbei kann vorteilhaft zur Beschleunigung des Trocknungsvorgangs und zur Einhaltung von niedrigen Temperaturen unter Vakuum (ca. 3.000-5.000 Pa (ca. 30-50 mbar)) gearbeitet werden. Es können Trocknungstemperaturen von bis zu 50°C gewählt werden, wobei die Temperatur während des Trocknungsvorganges in der Pelletmatrix aufgrund der Verdampfungsenthalpie der Flüssigkeit nicht über 30°C ansteigt.

Für konventionell getrocknete Pellets (Verfahrensvariante B) sind sol-gel-bildende Substanzen für die Matrix notwendig, die in Solform tropffähig sind und nach der Cryopelletierung bzw. nach dem Auftauen ein Gel ausbilden, das nach der Trocknung stabil ist. Ein Zusatz von Weichmachern beeinflußt die Matrixmasse hinsichtlich der Konsistenz. So hergestellte Pellets zeichnen sich durch eine besonders kostengünstige Herstellung aus, da der Verfahrensschritt der Lyophilisation nicht unbedingt notwendig ist.

Besonders vorteilhaft lassen sich ohne Zusatz von weiteren Emulgatoren lipophile Wirkstoffe z.B. mit Ultraschallhomogenisatoren bei Verwendung von Gelatinesorten und Kollagenhydrolysaten mit hohen Molekulargewichten vor der Weiterverarbeitung in stabile Emulsionen bzw. Mikroemulsionen verarbeiten.

Lipophile/ölige Wirkstoffe können z.B. sein: Knoblauchöl, Lebertran, Vitamin E und weitere fettlösliche Vitamine, Johanniskrautöl, Lecithin, Wacholderöl, Omega-3-Fettsäuren, Nachtkerzenöl, ätherische Öle etc. Bei Pflanzenextrakten, deren Wirkkomponenten sowohl hydrophile als auch lipophile Eigenschaften zeigen, werden die lipophilen Anteile zunächst in der Matrixmasse emulgiert und die wasserlöslichen Bestandteile in der hydrophilen Matrixmasse aufgelöst und anschließend cryopelletiert.

Bedingt durch die erhöhte Viskosität des Matrixsystems können suspendiert vorliegende Wirkstoffe durch einfaches Rühren an der Sedimentation gehindert und gleichmäßig dosiert werden. Temperaturempfindliche Arzneistoffe werden vorteilhaft lyophilisiert.

Die Verarbeitung der in den Unteransprüchen angegebenen besonderen Ausbildungsformen der Erfindung wie z.B. Formulierungen mit kontrollierter Freigabe bzw. Resorptionsverbesserung, Mikro- und Nanoverkapselung, Ausfällungen, Konjugatbildung, Filmüberzüge und die Herstellung von Pellets mit bioadhäsiven Eigenschaften erfolgt sinngemäß der Beschreibung und in Abstimmung auf den jeweiligen Wirkstoff.

Das erfindungsgemäße Verfahren selbst ist gegenüber dem Stand der Technik insgesamt gesehen wartungsarm und wirtschaftlich durchzuführen. Die an sich einfach durchzuführende Cryopelletierung ermöglicht auf überraschende Weise den Stand der Technik deutlich zu überragen.

Zur Durchführung des erfindungsgemäßen Verfahrens genügt es im einfachsten Fall, eine wäßrige Gelatinelösung mit einer Gelatinesorte der bezeichneten Spezifikation herzustellen, darin das Nifedipin bzw. das Dihydropyridinderivat in fein kristalliner Form homogen zu suspendieren, und das System über eine geeignete Dosiervorrichtung in ein Tauchbad mit flüssigem Stickstoff zu tropfen. Die auf diese Weise geformten, tiefgefrorenen Pellets werden anschließend durch Lyophilisation in den trockenen Zustand überführt.

Im Rahmen der vorliegenden Erfindung hat sich vorteilhaft gezeigt, daß feindisperse Dihydropyridin-Fällungen auch durch Präzipitation aus einer Lösung des Dihydropyridins in einem mit Wasser mischbaren und pharmazeutisch akzeptablen organischen Lösungsmittel, wie z. B. Alkohol, direkt in der Gelatinelösung erzeugt werden können. Nach Entfernen des Alkohols (z. B. durch Evaporation) verfährt man analog zu der beschriebenen Verfahrensweise, um die erfindungsgemäßen Formkörper herzustellen.

Für die bereits erwähnten Kombinationspräparate können Dihydropyridinderivate beispielsweise mit beta-Sympathicolytica oder Diuretika kombiniert werden.

Im Falle von optisch aktiven Substanzen lassen sich sowohl deren Racemate, wie auch die enantiomerreinen Komponenten und Mischungen davon einsetzen.

Bedingt durch die große Variationsbreite der Erfindung können in den beschriebenen Matrixmassen alle Arzneistoffe enthalten sein, sofern sie keine Inkompatibilitäten mit den einzelnen Bestandteilen der Rezepturmassen zeigen. Der Begriff Arzneistoff wird dabei erfindungsgemäß wie folgt definiert:

Arzneistoffe können synthetischen oder natürlichen Ursprungs sein, sowohl chemisch einheitliche Substanzen oder Substanzgemische sein, als auch Kombinationen aus verschiedenen pharmakologisch wirksamen Komponenten. Ferner soll der Arzneistoffbegriff aber auch Phytopharmaka und Pflanzenextrakte allgemein umfassen und schließlich auch Hormone, Vitamine und Enzyme mit einbeziehen.

Auch enantiomerreine Wirkstoffe oder Pseudoracemate sind erfindungsgemäß geeignet.

Weiterhin können Wirkstoffe aus dem Bereich der diätetischen Lebensmittel (health care) sowie aus dem Bereich der Kosmetik verwendet werden.

Im Falle für die Erfindung geeigneter Arzneistoffe besteht keinerlei Begrenzung bzgl. der Indikationsgruppen. Im folgenden werden beispielhaft Indikationsgruppen und einige zugehörige Vertreter genannt:

1. starke Analgetika, z.B. Morphin, Dextropropoxyphen, Pentazocin, Pethidin, Buprenorphin;

2. Antirheumatika/Antiphlogistika (NSAR), z.B. Indometacin, Diclofenac, Naproxen, Ketoprofen, Ibuprofen, Flurbiprofen, Acetylsalicylsäure, Oxicame;

3. beta-Sympathicolytica, z.B. Propranolol, Alprenolol, Atenolol, Bupranolol, Salbutamol;

4. Steroidhormone, z.B. Betamethason, Dexamethason, Methylprednisolon, Ethinylestradiol, Medroxyprogesteron, Prednison, Prednisolon;

5. Tranquillizer, z.B. Oxazepam, Diazepam, Lorazepam;

6. alpha-Sympathicolytica, z.B. Ergotamin, Dihydroergotamin, Dihydroergotoxin;

7. Hypnotika und Sedativa, z.B. Secbutabarbital, Secobarbital, Pentobarbital, Doxylamin, Diphenhydramin;

8. tricyclische Antidepressiva, z.B. Imipramin, Nortriptylin, Clomipramin, Amitryptilin;

9. Neuroleptika, z.B. Chlorprothixen, Chlorpromazin, Haloperidol, Triflupromazin;

10. Gichtmittel, z.B. Benzbromaron, Allopurinol;

11. Antiparkinsonmittel, z.B. Levodopa, Amantadin;

12. Koronartherapeutika oder Calciumantagonisten, z.B. Nifedipin u.a. Dihydropyridinderivate; Salpetersäureester wie Glycerintrinitrat, Isosorbidmononitrat und Isosorbiddinitrat; Verapamil, Gallopamil, Molsidomin;

13. Antihypertensiva, z.B. Clonidin, Methyldopa, Dihydralazin, Diazoxid;

14. Diuretika, z.B. Mefrusid, Hydrochlorothiazid, Furosemid, Triamteren, Spironolacton;

15. orale Antidiabetika, z.B. Tolbutamid, Glibenclamid;

16. Chemotherapeutika oder Antibiotika, z.B. Penicilline wie Phenoxymethylpenicillin, Amoxycillin, Ampicillin, Pivampicillin, Bacampicillin, Dicloxacillin, Flucloxacillin; Cephalosporine wie Cefalexin, Cefaclor; Gyrasehemmstoffe wie Nalidixinsäure, Ofloxacin, Norfloxacin; Erythromycin, Lincomycin, Tetracyclin, Doxycyclin, Trimethoprim, Sulfamethoxazol, Chloramphenicol, Rifampicin;

17. Lokalanästhetika, z.B. Benzocain;

18. ACE-Hemmstoffe, z.B. Enalapril, Captopril;

19. Mukolytika, z.B. Acetylcystein, Ambroxol, Bromhexin;

20. Antiasthmatika, z.B. Theophyllin;

21. Mineralstoffpräparate, z.B. Magnesium-, Calcium-, Kaliumsalze, Eisenpräparate;

22. Neurotropika, z.B. Piracetam;

23. Ulcustherapeutika, z.B. Cimetidin, Pirenzepin;

24. Provitamine und Vitamine, z.B. Biotin, Cyanocobalamin, Ergocalciferol, Ascorbinsäure, Thiamin, Pyridoxin, alpha-Tocopherol, Retinol, beta-Carotin;

25. Peptidarzneistoffe, z.B. Insulin, Interferone;

26. Digitalisglykoside, z.B. Digitoxin, Digoxin;

27. Antiemetika, z.B. Metoclopramid;

28. Enzyme, z.B Plasmin, Desoxiribonuklease;

29. Antiarrhytmika, z.B. Prajmalin;

30. Antiepileptika, z.B. Phenytoin;

31. Antikoagulantia, z.B. Phenprocoumon;

32. Spasmolytika, z.B. Papaverin;

33. Antimykotika, z.B. Clotrimazol;

34. Hormone, z.B. Calcitonin;

35. Venentherapeutika, z.B. Aescin;

36. Immunsuppresiva, z.B. Ciclosporin;

37. Tuberkulostatika, z.B. Rifampicin;

38. Virustatika, z.B. Aminoadamantan;

39. Zytostatika, z.B. Methotrexat;

40. Impfstoffe, z.B. Poliomyelitis-Lebendimpfstoff;

41. Phytopharmaka, z.B. Gingko biloba Extrakt;

42. Stoffe zur Behandlung von AIDS, wie z.B. Renin-Antagonisten.

43. Calciumantagonisten, wie Dihydropyridinderivate, insbesondere Nifedipin, Nitrendipin oder Nisoldipin.

Gegenüber dem Stand der Technik lassen sich besonders vorteilhaft Wirkstoffe mit schlechter Vertäglichkeit oder problematischer Bioverfügbarkeit, sowie licht-, oxidations-, hydrolyse- und temperaturempfindliche Stoffe wie z.B. schwerlösliche Arzneistoffe, Peptide, Naturstoffe, Enzyme, Vitamine etc. zu Arzneiformen erfindungsgemäß verarbeiten.

Um die physiologischen Hintergründe der Resorption von Arzneistoffen im allgemeinen und die verbesserte Resorptionsquote der erfindungsgemäßen Pelletformulierungen ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:

a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basisische Arzneistoffe bevorzugt im Darm resorbiert.

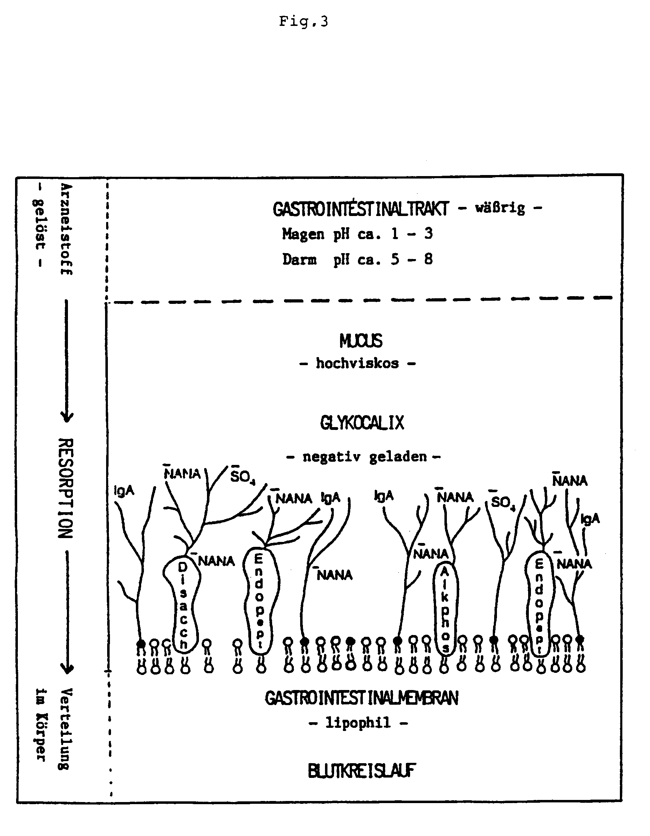

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Fig. 3), nämlich

- durch die Mucus-Schicht und eine wässerige, daran anhärierende Schicht

- die Zellmembranen der intestinalen Epithelzellen mit der daran kovalent gebundenen Glykocalix sowie

- die sogenannten "Tight Junctions", die die Epihelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand - durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Muscus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminsäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Formkörper geben erstmalig die technische Lehre, ein System zu bilden, mit dem diese vorgenannten Resorptionshindernisse zu überwinden sind.

Hydrophile Makromoleküle, insbesondere Gelatine, sind amphiphile Substanzen, die je nach pH-Wert unterschiedliche Ladungszustände aufweisen. Erfindungsgemäß kann nun das hydrophile Makromolekül in den erfindungsgemäßen Systemen so ausgewählt werden, bzw. der pH-Wert der Formulierung so abgestimmt werden, daß sich im physiologischen Milieu ein positiver Ladungszustand ergibt. Damit läßt sich zumindest eine Teilneutralisation der negativen Oberflächenladungen der Glycocalix erreichen. Dieses Neutralisationsphänomen kann durch bioadhäsive Eigenschaften des hydrophilen Makromoleküls, insbesondere Gelatine noch verstärkt wirksam werden.

Da gelöste Arzneistoffmoleküle nun die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption liefern.

Die Verwendung der erfindungsgemäßen Pulver, Granulate oder Pellets als Formkörper kann z.B. über übliche Dosiersysteme in Hartgelatinekapseln oder als Granluat in Beutel erfolgen. Durch die gute Rieselfähigkeit bzw. annähernd runde Form der Granulate läßt sich eine gute Dosierbarkeit gewährleisten. Bei Verwendung von Pellets ist die dichteste Kugelpackung in genauer Abstimmung des Schüttvolumens auf die Kapselgröße möglich, woraus eine Verbesserung der Dosiergenauigkeit beim Abfüllvorgang resultiert. Darüberhinaus kann durch die entsprechende Auswahl einer bestimmten Pelletgröße auf den Zusatz von Füllstoffen verzichtet werden.

Die Pellets mit einer Größe von 2-12 mm können erfindungsgemäß für eine neue einzeldosierte bukkale, nasale oder perorale Arzneiform verwendet werden. Peroral eingesetzte Pellets sind leicht schluckbar und können in Gläsern mit Dosierspendern umweltschonend abgegeben werden. Bei bukkaler und nasaler Verwendung eignen sich Formkörper-Pellets mit bioadhäsiven Eigenschaften.

Pulver, Granulate oder Pellets -als Formkörper- aus Matrixmassen, die sich in kaltem Wasser schnell und vollständig auflösen, können -in Beuteln abgefüllt- als Instantzubereitungen für den pharmazeutischen oder diätetischen Bereich (health care) verwendet werden.

Überraschenderweise können unter Ausnutzung der bioadhäsiven Eigenschaften der Sol-Gel-Bildner, insbesondere Gelatine, mit den erfindungsgemäßen Formkörpern bukkale und nasale Formulierungen bzw. Arzneiformen mit pH-gesteuerter Freigabe zur Anwendung kommen.

Eine weitere Verwendung dieser speziellen Granulate oder Pellets als Formkörper ist durch ihre direkte Verpreßbarkeit zu Tabletten gegeben. Die so erhaltenen Tabletten zeigen, bei geringer Friabilität und hoher Bruchfestigkeit, erstaunlicherweise eine völlige Auflösung innerhalb von 5 Minuten, z.B. 2 Minuten, gemessen nach üblichen Testmethoden (z.B. Dissolutiontestapparatur gemäß USP). Überraschenderweise bleiben die guten Auflösungseigenschaften der Gerüstmatrix auch nach dem Komprimieren erhalten. Die Tabletten lösen sich ohne vorgelagerten Zerfall direkt auf. Im Gegensatz dazu zerfallen aus herkömmlichen Granulaten verpreßte Tabletten zuerst immer in Granulatteilchen, die sich erst anschließend auflösen.

Die Tablettenherstellung aus erfindungsgemäßen, gefriergetrockneten Formkörpern ist beispielsweise bei der Konzipierung einer Arzneiform für temperaturempfindliche Wirkstoffe von Bedeutung. Solche Arzneistoffe erfordern wegen ihrer Empfindlichkeit (z.B. Hitzeinaktivierung usw.) besonders schonende Verarbeitungsprozesse, die vorteilhafterweise durch das erfindungsgemäße Verfahren sehr leicht und einfach sichergestellt werden können.

Der Anwendungsbereich für die erfindungsgemäßen Formkörper ist selbstverständlich nicht nur auf pharmazeutische Zwecke beschränkt. Einsatzgebiete können auch auf biotechnologischem (Cryokonservierung von Enzymen oder Bakterien, fertige Nährböden in getrockneter Form etc.) und auf kosmetischem Gebiet liegen (Verarbeitung von Planzenextrakten wie z.B. Aloe vera zu Pellets bietet den Vorteil einer idealen, trockenen Transportform für den feuchteempfindlichen Extrakt und zugleich ist das natürlich aufgebaute Matrixsystem als Bestandteil für Salben und Cremes besonders geeignet).

Bedingt durch die vielfältigen Variations- und Kombinationsmöglichkeiten der erfindungsgemäßen Formkörper läßt sich die Freisetzung von Arzneistoffen in allen angegebenen Verwendungszwecken in weiten Grenzen modulieren.

Die folgenden Beispiel sollen die Erfindung näher erläutern:

Beispiel 1:

Arzneistoff: Benzocain
Rezeptur der zu verarbeitenden Grundmasse:
210 g Gelatine 170 Bloom

50 g Dextran (Molekulargewicht ca. 10000)
29 g Saccharose
1 g Pfefferminzaroma
710 g destilliertes Wasser
1000 g

Das Gelatinepulver wird mit dem Pfefferminzaroma gemischt, das Wasser, das bereits das Dextran sowie die Saccharose gelöst enthält zugegeben und nach Vorquellung bei 50°C geschmolzen. In dieser Lösung werden 10 g mikronisiertes Benzocain unter Ultrabeschallung suspendiert.

Anschließend entlüftet man im Vakuum. Über die Cryopel®-Dosiervorrichtung tropft man in ein Tauchbad mit flüssigem Stickstoff und formt damit Pellets.

Die schockgefrosteten, runden Pellet-Formkörper werden in einer Gefriertrocknungsanlage mit einer Primärtrocknung bei -50°C und 5 Pa (0,05 mbar) und einer Sekundärtrocknung bei 22°C getrocknet.

78% der Pellets liegen im Größenbereich von 0,8-1 mm vor. Die getrockneten Pellets werden auf einer Exzenterpresse direkt zu einer Lutschtablette mit einem durchschnittlichen Benzocaingehalt von 5 mg verpreßt.

Beispiel 2:

Arzneistoff: Kaliumchlorid
Rezeptur der zu verarbeitenden Grundmasse:
625 g Kollagenhydrolysat (Molekulargewicht 2.000-3.000 D)
50 g Citronensäure
2325 g destilliertes Wasser
3000 g

Das Kollagenhydrolysat und die Citronensäure werden unter Rühren im Wasser gelöst. In dieser Lösung werden 190 g Kaliumchlorid gelöst.

Nach Entschäumen im Vakuum tropft man über die Cryopel®-Dosiervorrichtung in ein Tauchbad mit flüssigem Stickstoff und formt damit Pellets von durchschnittlich 4 mm Größe.

Durch anschließende Gefriertrocknung wird das Wasser wie in Beispiel 1 entzogen.

Die Pellets werden in luftdichte Beutel verpackt, entsprechend einer Einzeldosierung von 1 g Kaliumionen.

Der Inhalt eines Beutels löst sich in Wasser von Raumtemperatur innerhalb von 30 sec vollständig auf.

Beispiel 3:

Arzneistoff: Phenoxymethylpenicillin-Kalium
Rezeptur der zu verarbeitenden Grundmasse:
200 g Dextran (Molekulargewicht ca. 60000)
200 g Kollagenhydrolysat (Molekulargewicht 2.000-3.000)
5 g Orangenaroma
250 g Mannit
100 g Saccharose
destilliertes Wasser ad 2500g

Die Bestandteile werden gemischt und in dem Wasser gelöst. 100 g Phenoxymethylpenicillin-Kalium werden in dieser Lösung unter Rühren gelöst.

Nach Entschäumen im Vakuum tropft man über die Cryopel®-Dosiervorrichtung in ein Tauchbad mit flüssigem Stickstoff und formt damit Pellets. Durch anschließende Gefriertrocknung wird das Wasser entzogen.

2,31 g der getrockneten Pellets (entsprechend einem durchschnittlichen Gehalt an Phenoxymethylpenicillin-Kalium von 270 mg) und werden - in Einzelbeutel eingesiegelt - als Instanttrinklösung verwendet.

Beispiel 4:

Beispiel für eine Matrixmasse aus Gelatine und Weichmacher, in der zu verarbeitender Arzneistoff gelöst werden kann.

| Gelatine 150 Bloom | 2,6 kg |
|---|---|
| Sprühgetrocknetes Sorbitol | 1,0 kg |
| Dihydrocodeinhydrogentartrat | 0,1 kg |
| Wasser | 6,3 kg |

Der Wirkstoff wird in 1 kg Wasser unter Rühren vollständig gelöst. Das Gelatinegranulat wird in der verbleibenden Menge Wasser vorgequollen, bei 40°C aufgelöst und danach unter Rühren Sorbitol und die Wirkstofflösung zugegeben. Nach Schmelzen der Gelatine und homogenisieren der Lösung werden wie in Beispiel 1 beschrieben Pellets durch Eintropfen der Masse in flüssigen Stickstoff hergestellt. Die Pellets werden auf übliche Weise bei Temperaturen zwischen 20°C bis 40°C getrocknet und anschließend in opake Hartgelatinesteckkapseln mit einem durchschnittlichen Gehalt von 10 mg Dihydrocodeintartrat abgefüllt. Im Dissolutiontest (Apparatur nach USP XXI, 500 ml Wasser, 37°C, 50 UpM) setzt die Arzneiform 70% des Wirkstoffs in 4,5 Minuten frei.

Die erhaltenen Pellets sind durchsichtig klar und glänzend.

Beispiel 5:

Beispiel für eine Matrixmasse aus Gelatine und Weichmacher, in der der Arzneistoff emulgiert vorliegt.

| Gelatine 210 Bloom | 2,6 kg |
|---|---|
| Glycerin (85%ig) | 1,25 kg |
| a-Tocopherolacetat | 0,25 kg |
| Wasser | 6,9 kg |

Die pulverförmige Gelatine wird 40 Minuten in kaltem Wasser vorgequollen und anschließend bei 50°C gelöst. Mit einem Ultraschallhomogenisator wird der Wirkstoff bei 50°C in der Gelatinelösung emulgiert. Die Öl in Wasser Emulsion wird anschließend mit Glycerin vermischt und cryopelletiert. Die erhaltenen Pellets werden wie in Beispiel 4 getrocknet. Die Pellets werden mit einem Gehalt von 25 mg alpha-Tocopherolacetat in opake Hartgelatinesteckkapseln dosiert.

Die erhaltenen Pellets sehen opak undurchsichtig und glänzend aus.

Beispiel 6:

Beispiel für eine Matrixmasse aus Gelatine und Weichmacher, in der der Arzneistoff suspendiert werden kann.

| Gelatine 250 Bloom | 2,5 kg |
|---|---|
| Glycerin (85%ig) | 1,0 kg |
| Dexamethason, mikronisiertes Pulver | 0,025 kg |
| Wasser | 4,0 kg |

Die Weichgelatinemasse wird in 1 kg Wasser vorgequollen und nach Zugabe des restlichen Wassers bei 50°C aufgelöst. Der Wirkstoff wird unter Rühren in dieser Lösung homogen verteilt und anschließend wird die Lösung mit dem Glycerin vermischt. Die erhaltene Suspension wird cryopelletiert. Nach üblicher Trocknung werden die Pellets in Hartgelatinesteckkapseln mit einem Steroidgehalt von 0,5 mg abgefüllt.

Die erhaltenen Pellets sind durchsichtig und glänzend.

Beispiel 7:

Beispiel für eine einzeldosierte Arzneiform.
Ansatz:
0,8 kg Gelatine 250 Bloom
0,8 kg sprühgetrocknetes Sorbitol
0,8 kg Acetylsalicylsäure
1,6 kg Wasser

Das Gelatinegranulat wird in dem Wasser 30 Minuten vorgequollen und anschließend bei 70°C aufgelöst. Die Acetylsalicylsäure wird in der erhaltenen Lösung dispergiert und anschließend das Sorbitol zugegeben.

Die erhaltene Matrixmasse wird über die in Fig. 2 dargestellte Apparatur bei einer Temperatur der Düsen von 70°C in flüssigen Stickstoff eingetropft. Die schockgefrosteten Pellets werden unter Kühlung klassiert und weisen eine einheitliche Größe von 8 mm auf.

Die runden Formkörper werden in einen Dosierspender abgefüllt und können - je nach Indikation - individuell dosiert werden.

So hergestellte Pellets sind wohlschmeckend und steigern die Verträglichkeit, insbesondere bei der Herzinfarktprophylaxe.

Beispiel 8:

Herstellung einer Ibuprofen-Akutarzneiform auf Pelletbasis Demonstration der gesteigerten Bioverfügbarkeit.
Rezeptur:
400 g Ibuprofen USP XXII, mikronisiertes Pulver
400 g Gelatinepulver 220 Bloom
1400 g Wasser

Das Gelatinepulver wird in dem Wasser 45 Min. vorgequollen und anschließend bei 650°C aufgelöst. Das mikronisierte Ibuprofen wird in der Gelatinelösung homogen dispergiert und die entstandene Masse im Vakuum entlüftet.

Über die in Fig. 1 dargestellte Apperatur wird in flüssigen Stickstoff eingetropft und so Pellets geformt. Nach Trocknung bei Temperaturen zwischen 20°C bis 40°C werden die Pellets in Hartgelatinesteckkapseln mit einem Gehalt von 400 mg Ibuprofen abgefüllt.

Bei einer in vivo Humanstudie wurde die beschriebene Akutform gegen eine handelsübliche Ibuprofen-Akutformulierung, die 600 mg Ibuprofen (in mikronisierter Form) enthält, vergleichend getestet.

Es ergeben sich folgende durchschnittliche Plasmakonzentrations-Zeit-Werte, angegeben in µg Ibuprofen/ml Plasma.

| Zeit (h) | Formulierung aus Beispiel 8 | Vergleichspräparat |
|---|---|---|
| 1 | 27,5 | 5 |
| 2 | 35 | 19 |
| 2,5 | 37 | 23 |
| 3 | 35 | ....22 |
| 5 | 17,5 | 15 |
| 7 | 8 | .....6 |
| 9 | 5 | 5 |

Beispiel 9:

Herstellung einer Flurbiprofen-Akutarzneiform auf Pelletbasis, Demonstration der gesteigerten Bioverfügbarkeit.
Rezeptur:
50 g Flurbiprofen, mikronisiertes Pulver
50 g Gelatinepulver 220 Bloom
175 g Wasser

Das Gelatinepulver wird in dem Wasser 45 Min. vorgequollen und anschließend bei 60°C aufgelöst. Das mikronisierte Flurbiprofen wird in der Gelatinelösung homogen dispergiert und die entstandene Masse im Vakuum entlüftet.

Über die in Fig. 1 dargestellte Apparatur wird in flüssigen Stickstoff eingetropft und so Pellets geformt. Nach Trocknung bei Temperaturen zwischen 20°C bis 40°C werden die Pellets in Hartgelatinesteckkapseln mit einem Gehalt von 50 mg Flurbiprofen abgefüllt.

Bei einer in vivo Humanstudie wurde die beschriebene Akutform gegen eine handelsübliche Flurbiprofen-Akutformulierung, die 50 mg Flurbiprofen (in mikronisierter Form) enthält, vergleichend getestet.

Es ergeben sich folgende durchschnittliche Plasmakonzentrations-Zeit-Werte, angegeben in µg Flurbiprofen/ml Plasma.

| Zeit (h) | Formulierung aus Beispiel 9 | Vergleichspräparat |
|---|---|---|
| 0,5 | 6,5 | 2 |
| 1 | 8 | 6 |
| 2 | 6 | 4,5 |
| 3 | 6 | 3,5 |
| 5 | 4,5 | 2 |

Beispiel 10:

Arzneistoff: Nifedipin
Rezeptur der zu verarbeitenden Grundmasse:
300 g Kollagenhydrolysat
750 g Mannit
3950 g destilliertes Wasser

Das Kollagenhydrolysat und das Mannit werden unter Rühren in dem dest. Wasser gelöst. In dieser Lösung werden 100 g mikronisiertes Nifedipin, ggf unter Zusatz üblicher pharmazeutischer Hilfsstoffe homogen suspendiert. Nach Entschäumung unter Vakuum wird die Suspension durch Eintropfen bei Raumtemperatur über die Cryopel[R]-Dosiervorrichtung in ein Tauchbad mit flüssigem Stickstoff zu Pellets geformt.

Durch anschließende Gefriertrocknung wird das Wasser entzogen und man erhält, nach Klassierung, runde Formkörper mit einem durchschnittlichen Nifedipingehalt von 2 mg.

Diese Formkörper zerfallen in Wasser von Raumtemperatur (Dissolutiontestapparatur gemäß USP, Prüfmedium 100 ml Wasser, 23°C) innerhalb von 20 Sekunden vollständig und geben die enthaltene Nifedipinmenge frei.

Die getrockneten Formkörper werden in ein dunkel eingefärbtes Dosierbehältnis abgefüllt, in dem sie vor Lichtzutritt geschützt sind und aus dem die gewünschte Dosis entnommen werden kann.

Beispiel 11:

Die getrockneten Formkörper aus Beispiel 10 werden auf einer Exzenterpresse zu Tabletten mit einem durchschnittlichen Nifedipingehalt von 10 mg direkt verpreßt.

In einer Dissolutiontestapparatur nach USP (900 ml 0,1 N HCl, paddle, 75 Upm, 37°C) ergibt sich eine vollständige Tablettenauflösung und damit Wirkstofffreisetzung innerhalb von 5 Minuten.

Die Pellets aus Beispiel 10 können alternativ in opake Hartgelatinekapseln mit einem durchschnittlichen Nifedipingehalt von 5 mg abgefüllt werden.

Beispiel 12:

Die Rezeptur der zu verarbeitenden Grundmasse in Beispiel 10 wird wie folgt geändert:
300 g Kollagenhydrolysat
60 g Polyvinylpyrrolidon K 15
100 g Saccharose
2540 g destilliertes Wasser
Die weitere Arbeitsweise erfolgt analog Beispiel 10.

Die Beispiele stellen lediglich beispielhafte Ausführungsformen der vorliegenden Erfindung dar. Dem Fachmann steht es demnach frei ebenso sämtliche pharmazeutische, kosmetische oder andere erfindungsgemäße Formkörper wie Pulver, Granulate, im wesentlichen symmetrische Aggregate etc. bei Bedarf innerhalb des Umfangs der vorliegenden Erfindung zu verwenden oder herzustellen.

**Patentansprüche**

1.  Verfahren zur Herstellung von wenigstens einen Wirkstoff mit in vivo schlechter Resorbierbarkeit enthaltenden Pellets, dadurch gekennzeichnet, daß man

    a) einen Gerüstbildner aus hydrophilen Makromolekülen ausgewählt aus der Gruppe bestehend aus: Kollagen, Gelatine, fraktionierte Gelatine, Kollagenhydrolysate, Gelatinederivate, Pflanzenproteine, Pflanzenproteinhydrolysate, Elastinhydrolysate, in einem wäßrigen oder wäßrig-organischen Lösungsmittel löst,

    b) den Wirkstoff dispergiert und

    c) die erhaltene Mischung aus gelöstem Gerüstbildner und dispergiertem Wirkstoff in ein tiefkaltes inertes verflüssigtes Gas eintropft und so Pellets formt, und

    d) die so geformten Pellets durch Verdampfen oder Sublimieren des Lösungsmittels auf übliche Weise trocknet.

2.  Verfahren nach Anspruch 1, dadurch gekennzeichnet, daß man die Mischung in flüssigen Stickstoff eintropft.

3.  Verfahren nach Anspruch 1 und/oder 2, dadurch gekennzeichnet, daß man

    a) einen Gerüstbildner, ausgewählt aus der Gruppe bestehend aus: Gelatine, fraktionierte Gelatine, Kollagenhydrolysat, Gelatinederivaten; sowie deren Mischungen; in einem Lösungsmittel löst,

    b) in der Lösung ein Dihydropyridinderivat mit möglichst geringer Teilchengröße dispergiert,

    c) die Dispersion aus Gerüstbildner und Dihydropyridinderivat in flüssigen Stickstoff eintropft und so Pellets formt, und

    d) die so geformten Pellets durch Verdampfen oder Sublimieren des Lösungsmittels auf übliche Weise trocknet.

4.  Verfahren nach einem der Ansprüche 1 - 3, dadurch gekennzeichnet, daß man aus der Dispersion Tropfen in annähernd gleichmäßiger vorherbestimmter Form mittels eines Dosiersystems herstellt.

5.  Verfahren nach einem der Ansprüche 1 - 4, dadurch gekennzeichnet, daß man die Pellets gefriertrocknet.

6.  Verfahren nach einem der Ansprüche 1-5, dadurch gekennzeichnet, daß man die Dispersion aus Wirkstoff und Gerüstbildner mit einem zusätzlichen Gerüstbildner, ausgewählt aus der Gruppe bestehend aus: Albumine, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyacrylsäure und Polymere aus Methacrylsäure und Methacrylsäureestern, Celluloseacetatphthalat, Hydroxypropylmethylcellulosephthalat, azo-vernetzte Polymethacrylate, Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden, Galactomannanderivate wie Ethyl- oder Acetylgalactomannane, mit Adipinsäure vernetzte Polysaccharide, lipophile Substanzen wie abbaubare Mono-, Di-, und Triglyceride, erodierbare Fettalkohole; sowie deren Mischungen, versetzt.

7.  Verfahren nach einem der Ansprüche 1 - 6, dadurch gekennzeichnet, daß der Mischung aus Gerüstbildner und Wirkstoff Weichmacher und Geschmackskorrigentien, ausgewählt aus der Gruppe bestehend aus:
    Glycerol, Propylenglykol, Polyethylenglykol, Triacetin, Sorbitol, Sorbitangemische, Glucosesirup, und deren Mischungen,
    zugesetzt werden.

8. Verfahren nach einem der Ansprüche 1-7, dadurch gekennzeichnet, daß als Gerüstbildner Gelatine mit einem Maximum der Molekulargewichtsverteilung oberhalb $10^5$ D bei maximal 70°C mit dem Wirkstoff vermischt wird.

9. Verfahren nach einem der Ansprüche 1-8, dadurch gekennzeichnet, daß der Wirkstoff in gelöster, emulgierter, suspendierter, mikroverkapselter, nanoverkapselter, mikroemulgierter, feindisperser oder in konjugierter Form an dem hydrophilen Makromolekül, vorgelegt wird.

10. Verfahren nach Anspruch 1 und/oder 3, dadurch gekennzeichnet, daß man als Lösungsmittel Wasser einsetzt.

11. Wirkstoff enthaltendes Pellet,
**gekennzeichnet durch**
eine Dispersion wenigstens eines Wirkstoffs oder Wirkstoffgemisches mit in vivo schlechter Resorbierbarkeit in einer Matrix, die im wesentlichen einen Gerüstbildner aus hydrophilen Makromolekülen umfaßt, welche ausgewählt wurden aus der Gruppe bestehend aus:
Kollagen, Gelatine, fraktionierte Gelatine, Kollagenhydrolysate, Gelatinederivate, Pflanzenproteine, Pflanzenproteinhydrolysate, Elastinhydrolysate, sowie deren Mischungen, herstellbar durch das Verfahren nach einem der Ansprüche 1-10.

12. Pellet nach Anspruch 11, dadurch gekennzeichnet, daß das hydrophile Makromolekül ein thermoreversibler Sol-Gel-Bildner ist.

13. Pellet nach Anspruch 11 und/oder 12, dadurch gekennzeichnet, daß die Matrix einen zusätzlichen Gerüstbildner enthält, ausgewählt aus der Gruppe bestehend aus: Albumin, Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Dextran, Zucker, Glycin, Lactose, Mannit, Polyvinylpyrrolidon, Polyacrylsäure, Polymere aus Methacrylsäure, Polymere aus Methacrylsäureestern, Celluloseacetatphthalat, Hydroxypropylmethylcellulosephthalat, azovernetzte Polymethacrylate, Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden, Galactomannanderivate wie Ethyl- oder Acetylgalactomannane, mit Adipinsäure vernetzte Polysaccharide, lipophile Substanzen wie abbaubare Mono-, Di-, und Triglyceride, erodierbare Fettalkohole; sowie deren Mischungen.

14. Pellet nach Anspruch 13, dadurch gekennzeichnet, daß der Wirkstoff ein Dihydropyridinderivat ist und der Gerüstbildner hydrophile Makromoleküle umfaßt, ausgewählt aus der Gruppe bestehend aus: Gelatine, fraktionierte Gelatine, ein Gelatinederivat, Kollagenhydrolysat, oder deren Mischung; und der zusätzliche Gerüstbildner ausgewählt ist aus der Gruppe bestehend aus: Dextran, Zucker, Glyzin, Lactose, Sorbitol, Mannit, Polyvinylpyrrolidon, sowie deren Mischungen.

15. Pellet nach einem der Ansprüche 11-14, dadurch gekennzeichnet, daß der Gehalt der Matrix an zusätzlichen Gerüstbildnern weniger als etwa 50 Gew.-% beträgt.

16. Pellet nach Anspruch 11 gekennzeichnet durch einen pharmazeutisch akzeptablen Hilfs- oder Trägerstoff für die Matrix.

17. Pellet nach Anspruch 11, dadurch gekennzeichnet, daß es als im wesentlichen symmetrischer Festkörper, Aggregat oder als Mikropellet vorliegt.

18. Pellet nach Anspruch 11, dadurch gekennzeichnet, daß es als Lyophilisat vorliegt.

19. Pellet nach Anspruch 11, dadurch gekennzeichnet, daß
es schnellauflösend ist; und daß
die Matrix ein hydrophiles Makromolekül umfaßt, ausgewählt aus der Gruppe bestehend aus:
Pflanzenproteine, Pflanzenproteinhydrolysate, Elastinhydrolysate, Kollagenhydrolysate, kaltwasserlöslichen Gelatinen, Gelatinederivate; mit einem Maximum der Molekulargewichtsverteilung unterhalb $10^5$ D.

20. Pellet nach Anspruch 11, dadurch gekennzeichnet, daß die Matrix Weichmacher und Geschmackskorrigentien, ausgewählt aus der Gruppe bestehend aus:
Glycerol, Propylenglykol, Polyethylenglykol, Triacetin, Sorbitol, Sorbitangemische, Glucosesirup; und deren Mischungen enthält.

**21.** Pellet nach einem der Ansprüche 12-20, dadurch gekennzeichnet, daß der Sol-Gel-Bildner eine Gelatine mit einem Maximum der Molekulargewichtsverteilung oberhalb $10^5$ D ist.

**22.** Pellet nach Anspruch 11, dadurch gekennzeichnet, daß der Wirkstoff ausgewählt ist aus der Gruppe bestehend aus:
Arzneistoffen synthetischen oder natürlichen Ursprungs, Kosmetika, vorbeugenden Mitteln (health care), Enzymen, Mikroorganismen.

**23.** Pellet nach Anspruch 22, dadurch gekennzeichnet, daß der Wirkstoff ein Dihydropyridinderivat ist.

**24.** Pellet nach Anspruch 23, dadurch gekennzeichnet, daß das Dihydropyridinderivat ausgewählt ist aus der Gruppe bestehend aus: Nifedipin, Nitrendipin oder Nisoldipin

**25.** Pellet nach Anspruch 22, dadurch gekennzeichnet, daß der Wirkstoff gelöst, suspendiert, feindispers, emulgiert oder mikroemulgiert oder in Form von Liposomen vorliegt.

**26.** Pellet nach einem der Ansprüche 22-25, dadurch gekennzeichnet, daß der Wirkstoff als Konjugat mit dem hydrophilen Makromolekül vorliegt.

**27.** Pellet mit kontrollierter Freisetzung nach einem der Ansprüche 11-26, dadurch gekennzeichnet, daß die Matrix einen Schmelzbereich zwischen etwa 35°C und 40°C aufweist.

**28.** Pellet mit kontrollierter Freisetzung nach Anspruch 27, dadurch gekennzeichnet, daß die Matrix mit einem pharmazeutisch akzeptablen Härtungsmittel, ausgewählt aus der Gruppe bestehend aus: Aldosen, Citral, vernetzt vorliegt.

**29.** Pellet mit kontrollierter Freisetzung nach Anspruch 27 und/oder 28, dadurch gekennzeichnet, daß der Formkörpern ein zusätzliches hydrophiles Makromolekül als Gerüstbildner oder Filmüberzug enthält, das ausgewählt ist aus der Gruppe bestehend aus: Alginate, Alginat-Calciumphosphate, Pektine, Agar-Agar, Poly- und Methacrylsäurederivate, Cellulosederivate, Celluloseacetatphthalat, Hydroxypropylmethylcellulosephthalat, azo-vernetzte Polymethacrylate, Polyurethan-Zucker-Copolymere, wobei als Zuckerkomponente insbesondere oligomere Galactomannane oder Galactomannanderivate geeignet sind, die dann mit aliphatischen Diisocyanaten vernetzt werden, Galactomannanderivate wie Ethyl- oder Acetylgalactomannane, mit Adipinsäure vernetzte Polysaccharide, lipophile Substanzen wie abbaubare Mono-, Di-, und Triglyceride, erodierbare Fettalkohole.

**30.** Pellet nach Anspruch 22, dadurch gekennzeichnet, daß die Matrix umhüllt vorliegt.

**31.** Verwendung des Pellets nach Anspruch 11 zur Herstellung einer kosmetischen Zubereitung.

**32.** Verwendung des Pellets nach Anspruch 11 zur Herstellung einer Zubereitung, die ausgewählt ist aus der Gruppe bestehend aus:
pharmazeutischen, diagnostischen, analytischen Zubereitungen.

**33.** Verwendung des Pellets nach Anspruch 11 zur Herstellung einer Zubereitung, die ausgewählt ist aus der Gruppe bestehend aus:
nasalen, bukkalen, oralen sowie peroralen Heilmitteln.

**34.** Pharmazeutische Zubereitung, enthaltend Pellets nach Anspruch 11.

**35.** Kosmetische Zubereitung, enthaltend Pellets nach Anspruch 11.

**36.** Nahrungsmittelzubereitung (health care), enthaltend Pellets nach Anspruch 11.

**Claims**

**1.** Process for the production of pellets comprising at least one active compound having poor absorbability in vivo, characterized in that

22

a) a matrix former made of hydrophilic macromolecules selected from the group consisting of: collagen, gelatin, fractionated gelatin, collagen hydrolysates, gelatin derivatives, vegetable proteins, vegetable protein hydrolysates and elastin hydrolysates is dissolved in an aqueous or aqueous-organic solvent,

b) the active compound is dispersed and

c) the mixture of dissolved matrix former and dispersed active compound obtained is added in drop form to an intensely cold inert liquefied gas and pellets are thus formed, and

d) the pellets thus formed are dried by evaporating or subliming the solvent in a customary manner.

2. Process according to Claim 1, characterized in that the mixture is added in drop form to liquid nitrogen.

3. Process according to Claim 1 and/or Claim 2, characterized in that

a) a matrix former, selected from the group consisting of: gelatin, fractionated gelatin, collagen hydrolysate, gelatin derivatives; and mixtures thereof; is dissolved in a solvent,

b) a dihydropyridine derivative having a particle size which is as small as possible is dispersed in the solution, c) the dispersion of matrix former and dihydropyridine derivative is added in drop form to liquid nitrogen and pellets are thus formed, and

d) the pellets thus formed are dried by evaporating or subliming the solvent in a customary manner.

4. Process according to one of Claims 1 - 3, characterized in that drops in an approximately uniform predetermined shape are prepared from the dispersion by means of a metering system.

5. Process according to one of Claims 1 - 4, characterized in that the pellets are freeze-dried.

6. Process according to one of Claims 1 - 5, characterized in that the dispersion of active compound and matrix former is mixed with an additional matrix former selected from the group consisting of: albumins, agar-agar, gum arabic, pectins, tragacanth, xanthan, natural and modified starches, dextrans, dextrins, maltodextrin, chitosan, alginates, cellulose derivatives, polyvinyl alcohol, polyvinylpyrrolidone. polyacrylic acid and polymers of methacrylic acid and methacrylic acid esters, cellulose acetate phthalate, hydroxypropylmethylcellulose phthalate, azo-crosslinked polymethacrylates and polyurethane-sugar copolymers, a suitable sugar component in particular being oligomeric galactomannans or galactomannan derivatives which are then crosslinked with aliphatic diisocyanates, galactomannan derivatives such as ethyl- or acetylgalactomannans, polysaccharides crosslinked with adipic acid, lipophilic substances such as degradable mono-, di- and triglycerides, erodible fatty alcohols; and mixtures thereof.

7. Process according to one of Claims 1 - 6, characterized in that softening agents and flavour corrigents, selected from the group consisting of: glycerol, propylene glycol, polyethylene glycol, triacetin, sorbitol, sorbitan mixtures, glucose syrup, and mixtures thereof, are added to the mixture of matrix former and active compound.

8. Process according to one of Claims 1 - 7, characterized in that, as matrix former, gelatin having a maximum in the molecular weight distribution above $10^5$ D is mixed with the active compound at a maximum of 70°C.

9. Process according to one of Claims 1 - 8, characterized in that the active compound is initially present in dissolved, emulsified, suspended, microencapsulated, nanoencapsulated, microemulsified or finely disperse form or in a form conjugated to the hydrophilic macromolecule.

10. Process according to Claim 1 and/or Claim 3, characterized in that, as solvent, water is employed.

11. Pellet comprising active compound, characterized by a dispersion of at least one active compound or active compound mixture having poor absorbability in vivo in a matrix which essentially comprises a matrix former made of hydrophilic macromolecules which were selected from the group consisting of: collagen, gelatin, fractionated gelatin, collagen hydrolysates, gelatin derivatives, vegetable proteins, vegetable protein hydrolysates, elastin hydrolysates, and mixtures thereof, which can be prepared by the process according to one of Claims 1 - 10.

12. Pellet according to Claim 11, characterized in that the hydrophilic macromolecule is a thermoreversible sol-gel former.

13. Pellet according to Claim 11 and/or Claim 12, characterized in that the matrix contains an additional matrix former selected from the group consisting of: albumin, agar-agar, gum arabic, pectins, tragacanth, xanthan, natural and modified starches, dextrans, dextrins, maltodextrin, chitosan, alginates, cellulose derivatives, dextran, sugar, glycine, lactose, mannitol, polyvinylpyrrolidone, polyacrylic acid, polymers of methacrylic acid, polymers of methacrylic acid esters, cellulose acetate phthalate, hydroxypropylmethylcellulose phthalate, azo-crosslinked polymethacrylates and polyurethane-sugar copolymers, a suitable sugar component in particular being oligomeric galactomannans or galactomannan derivatives which are then crosslinked with aliphatic diisocyanates, galactomannan derivatives such as ethyl- or acetylgalactomannans, polysaccharides crosslinked with adipic acid, lipophilic substances such as degradable mono-, di- and triglycerides, erodible fatty alcohols; and mixtures thereof.

14. Pellet according to Claim 13, characterized in that the active compound is a dihydropyridine derivative and the matrix former comprises hydrophilic macromolecules selected from the group consisting of:
gelatin, fractionated gelatin, a gelatin derivative, collagen hydrolysate, or mixtures thereof; and the additional matrix former is selected from the group consisting of: dextran, sugar, glycine, lactose, sorbitol, mannitol, polyvinylpyrrolidone, and mixtures thereof.

15. Pellet according to one of Claims 11 - 14, characterized in that the content in the matrix of additional matrix formers is less than approximately 50% by weight.

16. Pellet according to Claim 11, characterized by a pharmaceutically acceptable auxiliary or excipient for the matrix.

17. Pellet according to Claim 11, characterized in that it is present as an essentially symmetrical solid or aggregate or as a micropellet.

18. Pellet according to Claim 11, characterized in that it is present as a lyophilizate.

19. Pellet according to Claim 11, characterized in that
it is rapidly dissolving; and in that
the matrix comprises a hydrophilic macromolecule selected from the group consisting of:
vegetable proteins, vegetable protein hydrolysates, elastin hydrolysates, collagen hydrolysates, cold water-soluble gelatins, gelatin derivatives; having a maximum in the molecular weight distribution below $10^5$ D.

20. Pellet according to Claim 11, characterized in that the matrix contains softening agents and flavour corrigents selected from the group consisting of: glycerol, propylene glycol, polyethylene glycol, triacetin, sorbitol, sorbitan mixtures, glucose syrup; and mixtures thereof.

21. Pellet according to one of Claims 12-20, characterized in that the sol-gel former is a gelatin having a maximum in the molecular weight distribution above $10^5$ D.

22. Pellet according to Claim 11, characterized in that the active compound is selected from the group consisting of: pharmaceutical substances of synthetic or natural origin, cosmetics, prophylactics (healthcare), enzymes, micro-organisms.

23. Pellet according to Claim 22, characterized in that the active compound is a dihydropyridine derivative.

24. Pellet according to Claim 23, characterized in that the dihydropyridine derivative is selected from the group consisting of: nifedipine, nitrendipine or nisoldipine.

25. Pellet according to Claim 22, characterized in that the active compound is present in dissolved, suspended, finely disperse, emulsified or microemulsified form or in the form of liposomes.

26. Pellet according to one of Claims 22-25, characterized in that the active compound is present as a conjugate with the hydrophilic macromolecule.

27. Pellet having controlled release according to one of Claims 11-26, characterized in that the matrix has a melting range of between approximately 35°C and 40°C.

**28.** Pellet having controlled release according to Claim 27, characterized in that the matrix is present crosslinked with a pharmaceutically acceptable hardening agent, selected from the group consisting of: aldoses and citral.

**29.** Pellet having controlled release according to Claim 27 and/or Claim 28, characterized in that the shaped article contains an additional hydrophilic macromolecule as a matrix former or film coating, which is selected from the group consisting of: alginates, alginate-calcium phosphates, pectins, agar-agar, poly- and methacrylic acid derivatives, cellulose derivatives, cellulose acetate phthalate, hydroxypropylmethylcellulose phthalate, azo-crosslinked polymethacrylates, polyurethane-sugar copolymers, a suitable sugar component in particular being oligomeric galactomannans or galactomannan derivatives which are then crosslinked with aliphatic diisocyanates, galactomannan derivatives such as ethyl- or acetylgalactomannans, polysaccharides crosslinked with adipic acid, lipophilic substances such as degradable mono-, di- and triglycerides, and erodible fatty alcohols.

**30.** Pellet according to Claim 22, characterized in that the matrix is present in coated form.

**31.** Use of the pellet according to Claim 11 for the production of a cosmetic preparation.

**32.** Use of the pellet according to Claim 11 for the production of a preparation which is selected from the group consisting of:
pharmaceutical, diagnostic and analytical preparations.

**33.** Use of the pellet according to Claim 11 for the production of a preparation which is selected from the group consisting of:
nasal, buccal, oral and peroral therapeutics.

**34.** Pharmaceutical preparation, comprising pellets according to Claim 11.

**35.** Cosmetic preparation, comprising pellets according to Claim 11.

**36.** Foodstuff preparation (healthcare), comprising pellets according to Claim 11.

**Revendications**

**1.** Procédé de production de pellets contenant au moins une substance active ayant une mauvaise capacité de résorption in vivo, caractérisé en ce que

a) on dissout dans un solvant aqueux ou aqueux-organique une matière formant la structure choisie entre des macromolécules hydrophiles du groupe comprenant :
le collagène, la gélatine, une gélatine fractionnée, des hydrolysats de collagène, des dérivés de gélatine, des protéines végétales, des hydrolysats de protéines végétales, des hydrolysats d'élastine,
b) on disperse la substance active et,
c) on verse goutte à goutte dans un gaz liquéfié inerte à basse température le mélange obtenu à partir de la matière dissoute formant la structure et de la substance active dispersée, en formant ainsi des pellets, et
d) on sèche de manière classique les pellets ainsi formés, par évaporation ou sublimation du solvant.

**2.** Procédé suivant la revendication 1, caractérisé en ce qu'on verse le mélange goutte à goutte dans de l'azote liquide.

**3.** Procédé suivant la revendication 1 et/ou la revendication 2, caractérisé en ce que :

a) on dissout dans un solvant une matière formant la structure, choisie dans le groupe comprenant la gélatine, une gélatine fractionnée, un hydrolysat de collagène, des dérivés de gélatine ; ainsi que leurs mélanges,
b) on disperse dans la solution un dérivé de dihydropyridine en particules de diamètre le plus petit possible,
c) on verse goutte à goutte dans de l'azote liquide la dispersion de la matière formant la structure et du dérivé de dihydropyridine et on forme ainsi des pellets, et
d) on sèche d'une manière classique les pellets ainsi formés, par évaporation ou sublimation du solvant.

**4.** Procédé suivant l'une des revendications 1 à 3, caractérisé en ce qu'on produit des gouttes ayant une forme prédéterminée à peu près régulière à partir de la dispersion, au moyen d'un système doseur.

5. Procédé suivant l'une des revendications 1 à 4, caractérisé en ce qu'on lyophilise les pellets.

6. Procédé suivant l'une des revendications 1 à 5, caractérisé en ce qu'on ajoute à la dispersion de substance active et de matière formant la structure, une autre matière formant la structure choisie dans le groupe comprenant :
l'albumine, l'agar-agar, la gomme arabique, la pectine, la gomme adragante, la gomme xanthane, des matières amylacées naturelles ainsi que modifiées, des dextranes, des dextrines, de la maltodextrine, du chitosane, des alginates, des dérivés de cellulose, un polymère d'alcool vinylique, une polyvinylpyrrolidone, un polymère d'acide acrylique et des polymères d'acide méthacrylique et d'esters d'acide méthacrylique, l'acétate-phtalate de cellulose, le phtalate d'hydroxypropylméthylcellulose, des polyméthacrylates azo-réticulés, des copolymères polyuréthanne-sucre, auquel cas on peut utiliser comme composant sucre en particulier des galactomannanes ou des dérivés de galactomannane oligomères, qui se réticulent ensuite avec des diisocyanates aliphatiques, des dérivés de galactomannane tels que l'éthyl- ou l'acétylgalactomannane, des polysaccharides réticulés avec de l'acide adipique, des substances lipophiles telles que des monoglycérides, diglycérides et triglycérides dégradables, des alcools gras pouvant être érodés ; ainsi que leurs mélanges.

7. Procédé suivant l'une des revendications 1 à 6, caractérisé en ce qu'on ajoute au mélange de la matière formant la structure et de la substance active, des plastifiants et des substances corrigeant le goût, choisis dans le groupe comprenant :
le glycérol, le propylène-glycol, un polyéthylène-glycol, la triacétine, le sorbitol, des mélanges de sorbitanne, du sirop de glucose et des mélanges de ces substances.

8. Procédé suivant l'une des revendications 1 à 7, caractérisé en ce qu'on mélange avec la substance active, comme matière formant la structure, de la gélatine ayant un maximum de répartition de poids moléculaire au-dessus de $10^5$ D à une température maximale de 70°C.

9. Procédé suivant l'une des revendications 1 à 8, caractérisé en ce que la substance active est préalablement mise en place sur la macromolécule hydrophile sous la forme dissoute, émulsionnée, mise en suspension, micro-encapsulée, nano-encapsulée, micro-émulsionnée, finement dispersée ou sous une forme conjuguée.

10. Procédé suivant la revendication 1 et/ou la revendication 3, caractérisé en ce qu'on utilise l'eau comme solvant.

11. Pellet contenant une substance active, caractérisé par
une dispersion d'au moins une substance active ou un mélange de substances actives de mauvaise capacité de résorption in vivo dans une matrice qui comprend principalement une matière formant la structure constituée de macromolécules hydrophiles qui ont été choisies dans le groupe comprenant :
le collagène, la gélatine, une gélatine fractionnée, des hydrolysats de collagène, des dérivés de gélatine, des protéines végétales, des hydrolysats de protéines végétales, des hydrolysats d'élastine ainsi que leurs mélanges, pouvant être produit par le procédé suivant l'une des revendications 1 à 10.

12. Pellet suivant la revendication 11, caractérisé en ce que la macromolécule hydrophile est un producteur sol-gel thermoréversible.

13. Pellet suivant la revendication 11 et/ou la revendication 12, caractérisé en ce que la matrice contient une matière additionnelle formant la structure, choisie dans le groupe comprenant : l'albumine, l'agar-agar, la gomme arabique, la pectine, la gomme adragante, la gomme xanthane, des substances amylacées naturelles ainsi que modifiées, des dextranes, des dextrines, la maltodextrine, le chitosane, des alginates, des dérivés cellulosiques, le dextrane, des sucres, la glycine, le lactose, le mannitol, la polyvinylpyrrolidone, un polymère d'acide acrylique, des polymères d'acide méthacrylique, des polymères d'esters d'acide méthacrylique, l'acétate-phtalate de cellulose, le phtalate d'hydroxypropylméthylcellulose, des polyméthacrylates azo-réticulés, des copolymères polyuréthanne-sucre, auquel cas on peut utiliser comme composant sucre, notamment des galactomannanes ou des dérivés de galactomannane oligomères qui sont réticulés ensuite avec des diisocyanates aliphatiques, des dérivés de galactomannane tels que des éthyl- ou acétylgalactomannanes, des polysaccharides réticulés avec l'acide adipique, des substances lipophiles telles que des monoglycérides, diglycérides et triglycérides dégradables, des alcools gras pouvant être érodés ; ainsi que leurs mélanges.

14. Pellet suivant la revendication 13, caractérisé en ce que la substance active est un dérivé de dihydropyridine et la matière formant la structure comprend des macromolécules hydrophiles choisies dans le groupe comprenant : la gélatine, une gélatine fractionnée, un dérivé de gélatine, un hydrolysat de collagène ou un mélange de ces matières ; et la matière additionnelle formant la structure est choisie dans le groupe comprenant : le dextrane, un sucre, la glycine, le lactose, le sorbitol, le mannitol, la polyvinylpyrrolidone, ainsi que des mélanges de ces matières.

15. Pellet suivant l'une des revendications 11 à 14, caractérisé en ce que la teneur de la matrice en matières additionnelles formant la structure s'élève à moins de 50 % en poids environ.

16. Pellet suivant la revendication 11, caractérisé par une substance auxiliaire ou un support acceptable du point de vue pharmaceutique pour la matrice.

17. Pellet suivant la revendication 11, caractérisé en ce qu'il est présent principalement comme corps solide symétrique, agrégat ou comme micropellet.

18. Pellet suivant la revendication 11, caractérisé en ce qu'il est présent sous forme de lyophilisat.

19. Pellet suivant la revendication 11, caractérisé en ce que :
il se dissout rapidement ; et en ce que
la matrice comprend une macromolécule hydrophile choisie dans le groupe comprenant :
des protéines végétales, des hydrolysats de protéines végétales, des hydrolysats d'élastine, des hydrolysats de collagène, des gélatines solubles à l'eau froide, des dérivés de gélatine, avec un maximum de répartition de poids moléculaire au-dessous de $10^5$ D.

20. Pellet suivant la revendication 11, caractérisé en ce que la matrice contient des plastifiants et des correcteurs de goût, choisis dans le groupe comprenant : le glycérol, le propylène-glycol, un polyéthylène-glycol, la triacétine, le sorbitol, des mélanges de sorbitanne, du sirop de glucose ; et des mélanges de ces composants.

21. Pellet suivant l'une des revendications 12 à 20, caractérisé en ce que le producteur sol-gel est une gélatine ayant un maximum de répartition de poids moléculaire au-dessus de $10^5$ D.

22. Pellet suivant la revendication 11, caractérisé en ce que la substance active est choisie dans le groupe comprenant :
des médicaments d'origine synthétique ou naturelle, des cosmétiques, des agents prophylactiques (health care), des enzymes, des micro-organismes.

23. Pellet suivant la revendication 22, caractérisé en ce que la substance active est un dérivé de dihydropyridine.

24. Pellet suivant la revendication 23, caractérisé en ce que le dérivé de dihydropyridine est choisi dans le groupe comprenant la nifédipine, la nitrendipine ou la nisoldipine.

25. Pellet suivant la revendication 22, caractérisé en ce que la substance active se présente à l'état dissous, en suspension, en fine dispersion, en émulsion ou en micro-émulsion ou sous forme de liposomes.

26. Pellet suivant l'une des revendications 22 à 25, caractérisé en ce que la substance active se présente comme conjugué avec la macromolécule hydrophile.

27. Pellet à libération contrôlée suivant l'une des revendications 11 à 26, caractérisé en ce que la matrice présente une plage de fusion entre environ 35°C et 40°C.

28. Pellet à libération contrôlée suivant la revendication 27, caractérisé en ce que la matrice se présente à l'état réticulé avec un agent durcissant acceptable du point de vue pharmaceutique choisi dans le groupe comprenant des aldoses et le citral.

29. Pellet à libération contrôlée suivant la revendication 27 et/ou la revendication 28, caractérisé en ce que le corps de forme contient une macromolécule hydrophile supplémentaire comme matière formant la structure ou comme film de revêtement, qui est choisie dans le groupe comprenant : alginates, alginate-phosphates de calcium, pectines, agar-agar, polymère d'acide méthacrylique et dérivés d'acide méthacrylique, dérivés de cellulose, acétate-phtalate de cellulose, phtalate d'hydroxypropylméthylcellulose, polyméthacrylatesazo-réticulés, copolymèrespolyuréthanne-sucre, auquel cas on peut utiliser comme composant sucre en particulier des galactomannanes ou des dérivés de galactomannane oligomères qui sont ensuite réticulés avec des diisocyanates aliphatiques, dérivés de galactomannane tels qu'éthyl- ou acétylgalactomannanes, polysaccharides réticulés avec l'acide adipique, substances lipophiles telles que mono-, di- et triglycérides dégradables, alcools gras pouvant être érodés.

30. Pellet suivant la revendication 22, caractérisé en ce que la matrice se présente à l'état enrobé.

31. Utilisation du pellet suivant la revendication 11 pour la production d'une préparation cosmétique.

32. Utilisation du pellet suivant la revendication 11 pour la production d'une préparation qui est choisie dans le groupe comprenant :

  des préparations pharmaceutiques, diagnostiques, analytiques.

33. Utilisation du pellet suivant la revendication 11 pour l'obtention d'une préparation qui est choisie dans le groupe comprenant :

  des médicaments à usage nasal, buccal, oral ainsi que peroral.

34. Préparation pharmaceutique contenant des pellets suivant la revendication 11.

35. Préparation cosmétique, contenant des pellets suivant la revendication 11.

36. Préparation alimentaire (health care) contenant des pellets suivant la revendication 11.

FIG. 1

FIG. 2

EP 0 621 777 B1

Fig.3